# EUROPÄISCHE PATENTSCHRIFT

(11) **EP 3 592 253 B1**
(45) Veröffentlichungstag und Bekanntmachung des Hinweises auf die Patenterteilung: **22.11.2023**
(21) Anmeldenummer: 18709555.9
(22) Anmeldetag: 07.03.2018
(51) Int. Cl.: A61B 17/15, A61B 90/00, A61B 34/20

(54) **MEDIZINISCHES SÄGESCHABLONENSYSTEM**
MEDICAL SAW JIG SYSTEM
SYSTÈME DE GABARIT DE SCIE À USAGE MÉDICAL

(30) Priorität: 07.03.2017 DE 102017104753
(43) Veröffentlichungstag der Anmeldung: 15.01.2020
(73) Patentinhaber: Aesculap AG, 78532 Tuttlingen (DE)
(72) Erfinder: NONNENMANN, Martin, 78573 Wurmlingen (DE); FIRMBACH, Franz-Peter, 78576 Emmingen-Liptingen (DE)
(74) Vertreter: Hoeger, Stellrecht & Partner Patentanwälte mbB
(86) Internationale Anmeldenummer: PCT/EP2018/055533
(87) Internationale Veröffentlichungsnummer: WO 2018/162518

(56) Entgegenhaltungen:
- DE-A1-102015 104 223

## Beschreibung

Die vorliegende Erfindung betrifft ein medizinisches Sägeschablonensystem umfassend eine Ausrichtvorrichtung zum Ausrichten einer medizinischen Sägeschablone relativ zu einem menschlichen oder tierischen Knochen, welche Ausrichtvorrichtung mindestens eine Befestigungselementaufnahme für ein in Knochen festlegbares Befestigungselement, ein erstes Kopplungsglied, welches mit einer Sägeschablone koppelbar oder gekoppelt ist, eine Abstandsänderungseinrichtung zum Verändern eines Abstands zwischen der Befestigungselementaufnahme und dem ersten Kopplungsglied und eine Winkeländerungseinrichtung zum Verändern eines Winkels zwischen der Befestigungselementaufnahme und dem ersten Kopplungsglied umfasst, wobei die Abstandsänderungseinrichtung ein um eine Abstandseinstellglieddrehachse verdrehbares Abstandseinstellglied umfasst und wobei die Winkeländerungseinrichtung ein um eine Winkeleinstellglieddrehachse verdrehbares Winkeleinstellglied umfasst.

Medizinische Sägeschablonensysteme der eingangs beschriebenen Art werden insbesondere eingesetzt, um Knochenflächen vor der Implantation von Gelenkendoprothesen zu präparieren. Beispielsweise werden derartige Schablonensysteme genutzt, um Tibia-und/oder Femur eines Patienten teilweise zu resezieren, um Knochenflächen zu präparieren, an denen Komponenten der Gelenkendoprothese festgelegt werden, beispielsweise durch Einschlagen in Knochenkavitäten, Festlegen mit Knochenzement und/oder Knochenbefestigungsmitteln wie beispielsweise Knochenschrauben oder Knochennägeln.

Um eine Sägeschablone, insbesondere eine Führungsöffnung derselben für ein Sägeblatt, in gewünschter Weise ausrichten zu können, um eine optimale Positionierung der Gelenkkomponenten sicherstellen zu können, lassen sich eine Höhe und eine Neigung der Führungsdurchbrechung der Sägeschablone mit der Ausrichtvorrichtung auf einfache Weise einstellen. Zum einen wird dies ermöglicht durch die Abstandsänderungseinrichtung, mit der beispielsweise das erste Kopplungsglied translatorisch bewegt werden kann, und zum anderen durch die Winkeländerungseinrichtung, mit der eine Neigung des ersten Kopplungsglieds, mit der Sägeschablone einstellbar ist.

Berücksichtigt man, dass bei der Implantation einer Kniegelenkendoprothese nur sehr wenig Raum zur Verfügung steht, ist es wünschenswert, das Sägeschablonensystem möglichst kompakt auszubilden. Außerdem soll dessen Handhabung für einen Operateur einfach und intuitiv möglich sein.

Aus der DE 10 2015 104 223 A1 ist ein medizinisches Instrument bekannt.

Es ist daher eine Aufgabe der vorliegenden Erfindung, ein medizinisches Sägeschablonensystem der eingangs beschriebenen Art so zu verbessern, dass es auf einfache Weise handhabbar ist.

Diese Aufgabe wird bei einem medizinischen Sägeschablonensystem der eingangs beschriebenen Art erfindungsgemäß dadurch gelöst, dass die Abstandseinstellglieddrehachse die Winkeleinstellglieddrehachse definiert.

Mit einer derartigen Ausrichtvorrichtung ist es insbesondere möglich, eine mit dieser gekoppelte Sägeschablone auf einfache Weise und hochpräzise auszurichten, wenn beispielsweise die Ausrichtvorrichtung mit einem Knochenbefestigungselement, das in die mindestens eine Befestigungselementaufnahme der Ausrichtvorrichtung eingreift, an einem Knochen festgelegt ist. Durch Verdrehen des Abstandeinstellglieds kann so das erste Kopplungsglied und eine mit diesem gekoppelte oder verbundene Sägeschablone beispielsweise in einer Höhe verstellt werden. Eine Neigung einer Führungsdurchbrechung der Sägeschablone kann insbesondere auf einfache Weise dadurch geändert werden, dass das Winkeleinstellglied verdreht wird. Beispielsweise lassen sich das Abstandseinstellglied und/oder das Winkeleinstellglied in Form von Einstellrädern ausbilden. Diese können beispielsweise mit Skalierungen versehen sein, um so für einen Operateur auf einfache Weise erkennbar zu machen, in welcher Weise er das erste Kopplungsglied beziehungsweise die Sägeschablone bewegt oder deren Neigung verstellt hat. Besonders einfach und kompakt ausbilden lässt sich das Sägeschablonensystem, weil die Abstandseinstellglieddrehachse die Winkeleinstellglieddrehachse definiert. Damit fallen beide Drehachsen zusammen. Diese sind hier mathematisch zu verstehen und nicht als mechanische Achsen wie beispielsweise Lagerachsen oder dergleichen.

Um eine möglichst individuelle Einstellung einer Position und/oder Neigung des ersten Kopplungsglieds zu ermöglichen, ist es vorteilhaft, wenn die Abstandsänderungseinrichtung und die Winkeländerungseinrichtung unabhängig voneinander betätigbar sind. Beispielsweise kann dies derart ermöglicht werden, dass das Abstandseinstellglied und das Winkeleinstellglied unabhängig voneinander verdrehbar angeordnet oder ausgebildet sind.

Für eine Handhabung des Sägeschablonensystems ist es vorteilhaft, wenn das Abstandseinstellglied in Form eines Einstellrads ausgebildet ist.

Ferner ist es günstig, wenn das Winkeleinstellglied in Form eines Einstellrads ausgebildet ist. Es lässt sich vom Operateur auf einfache Weise verdrehen zum Einstellen eines Neigungswinkels der Sägeschablone relativ zu einem Knochen, an dem das Sägeschablonensystem angeordnet ist.

Gemäß einer weiteren bevorzugten Ausführungsform der Erfindung kann vorgesehen sein, dass die Ausrichtvorrichtung einen Grundkörper umfasst, an welchem das Abstandseinstellglied und das Winkeleinstellglied verdrehbar gelagert oder gehalten sind. Einen solchen Grundkörper vorzusehen ermöglicht einen besonders kompakten Aufbau des Sägeschablonensystems. Insbesondere kann eine offene Bauweise des Grundkörpers vorgesehen werden, welche eine gute Reinigbarkeit des Sägeschablonensystems ermöglicht.

Günstig ist es, wenn dem Abstandseinstellglied ein Abstandseinstellgliedlagerelement zugeordnet ist und wenn dem Winkeleinstellglied ein Winkeleinstellgliedlagerelement zugeordnet ist. Mit diesen lassen sich das Abstandseinstellglied und das Winkeleinstellglied auf einfache und sichere Weisen am Grundkörper verdrehbar lagern oder halten.

Besonders einfach und kostengünstig ausbilden lässt sich das Sägeschablonensystem, wenn das Abstandseinstellgliedlagerelement und/oder das Winkeleinstellgliedlagerelement in Form eines Lagerstifts oder einer Schraube ausgebildet sind. Beispielsweise können das Abstandseinstellglied und das Winkeleinstellglied Durchbrechungen zum Aufnehmen des Lagerstifts oder einer Schraube aufweisen und mittels derselben am Grundkörper derart festgelegt werden, dass sie relativ zum Grundkörper noch verdrehbar sind. Insbesondere sind das Abstandseinstellgliedlagerelement und das Winkeleinstellgliedlagerelement derart angeordnet und ausgebildet, dass das Abstandseinstellglied und das Winkeleinstellglied lediglich verdrehbar um die Abstandseinstellglieddrehachse beziehungsweise die Winkeleinstellglieddrehachse gelagert sind, jedoch nicht axial parallel zu diesen verschoben werden können.

Auf einfache Weise lässt sich das Abstandseinstellglied am Grundkörper verdrehbar lagern, wenn das Abstandseinstellgliedlagerelement am Grundkörper oder am Abstandeinstellglied angeordnet oder ausgebildet ist.

Ferner ist es vorteilhaft, wenn das Winkeleinstellgliedlagerelement am Grundkörper oder am Winkeleinstellglied angeordnet oder ausgebildet ist. So lässt sich das Winkeleinstellglied auf einfache Weise verdrehbar am Grundkörper lagern.

Günstig ist es, wenn das Abstandseinstellglied und/oder das Winkeleinstellglied mindestens eine in radialer Richtung von der Abstandseinstellglieddrehachse weg weisende Ausnehmung und/oder mindestens einen in radialer Richtung von der Abstandseinstellglieddrehachse weg weisenden Vorsprung aufweisen. Die Ausnehmungen können insbesondere in Form von konkaven Vertiefungen ausgebildet sein, die eine Haptik für einen Operateur beim Betätigen, also beim Verdrehen des Abstandseinstellglieds beziehungsweise des Winkeleinstellglieds verbessern. Mindestens einen Vorsprung vorzusehen, welcher in radialer Richtung absteht, hat zudem den Vorteil, dass so ein Operateur beispielsweise auch gut erkennen kann, in welcher Richtung er das Abstandseinstellglied beziehungsweise das Winkeleinstellglied verdreht hat. Durch eine optionale Ausnehmung am mindestens einen Vorsprung kann die Adaption eines Hebels ermöglicht werden, wodurch die Haptik für den Operateur beim Betätigen noch weiter verbessert werden kann.

Gemäß einer weiteren bevorzugten Ausführungsform der Erfindung kann vorgesehen sein, dass die Abstandsänderungseinrichtung ein am Grundkörper verschiebbar gelagertes, eine Schiebeelementlängsachse definierendes Schiebeelement umfasst, welches mit dem Abstandseinstellglied direkt oder indirekt zusammenwirkend angeordnet oder ausgebildet ist derart, dass das Schiebeelement in Richtung der Schiebeelementlängsachse verschiebbar ist durch Verdrehen des Abstandseinstellglieds um die Abstandseinstellglieddrehachse. Besonders kompakt ausbilden lässt sich die Ausrichtvorrichtung, wenn das Abstandseinstellglied und das Schiebeelement direkt miteinander zusammenwirkend ausgebildet sind. So können aufwändige Getriebekonstruktionen vermieden werden.

Um eine definierte Orientierung der Sägeschablone bei einer Verschiebung des Schiebeelements sicherstellen zu können, ist es günstig, wenn das Schiebeelement am Grundkörper bezogen auf die Schiebeelementlängsachse unverdrehbar geführt ist.

Um eine Bewegung des Schiebeelements in definierter Weise vorgeben zu können, ist es vorteilhaft, wenn der Grundkörper eine Schiebeelementführung umfasst, in welcher das Schiebeelement beweglich geführt ist.

Auf einfache Weise ausbilden lässt sich der Grundkörper, wenn die Schiebeelementführung in Form einer Ausnehmung oder Durchbrechung ausgebildet ist.

Vorzugsweise weist die die Schiebeelementführung einen unrunden Querschnitt auf. So lässt sich auf einfache Weise eine Verdrehsicherung für das Schiebeelement relativ zum Grundkörper ausbilden, so dass dieses nicht um die Schiebeelementlängsachse verdreht werden kann.

Vorteilhaft ist es, wenn die die Abstandsänderungseinrichtung einen exzentrisch zur Abstandseinstellglieddrehachse angeordneten Schiebemitnehmer umfasst, wenn der Schiebemitnehmer am Abstandseinstellglied angeordnet oder ausgebildet ist und mit dem Schiebeelement beweglich in Eingriff steht oder wenn der Schiebemitnehmer am Schiebeelement angeordnet oder ausgebildet ist und mit dem Abstandseinstellglied beweglich in Eingriff steht. Diese vorgeschlagene Ausgestaltung ermöglicht auf einfache Weise ein direktes Zusammenwirken des Abstandseinstellglieds und des Schiebeelements über den Schiebemitnehmer.

Besonders einfach und kompakt ausbilden lässt sich die Ausrichtvorrichtung, wenn der Schiebemitnehmer in Form eines Stifts ausgebildet ist und wenn eine Stiftlängsachse des Stifts parallel oder im Wesentlichen parallel zur Abstandseinstellglieddrehachse verläuft.

Ferner ist es vorteilhaft, wenn der Schiebemitnehmer in eine korrespondierende Führungsausnehmung eintaucht zum Übertragen einer Antriebskraft vom Abstandseinstellglied auf das Schiebeelement. Beispielsweise kann die Führungsausnehmung am Schiebeelement angeordnet oder ausgebildet sein, wenn der Schiebemitnehmer am Abstandseinstellglied angeordnet oder ausgebildet ist. Die Führungsausnehmung kann insbesondere ausgebildet sein durch zwei parallel zueinander ausgerichtete Führungsstifte, die voneinander beabstandet sind, so dass der Schiebemitnehmer zwischen diese eingreifen kann. Ein Abstand zwischen den Führungsstiften entspricht vorzugsweise in etwa einem Durchmesser des Schiebemitnehmers, so dass hier eine möglichst spielfreie Kraftübertragung möglich ist.

Vorzugsweise verläuft die Schiebeelementlängsachse quer, insbesondere senkrecht, zur Abstandseinstellglieddrehachse. So lässt sich Ausrichtvorrichtung insbesondere derart ausbilden, dass eine Drehrichtung des Abstandseinstellglieds im Uhrzeigersinn ein Verschiebebewegung des Verschiebeelements in eine Richtung und eine Verdrehung des Abstandseinstellglieds entgegen dem Uhrzeigersinn eine Verschiebung des Schiebeelements in entgegengesetzter Richtung bewirken kann.

Ferner ist es vorteilhaft, wenn die Winkeländerungseinrichtung ein am Grundkörper um eine Schwenkachse verschwenkbar gelagertes Schwenkelement umfasst, welches mit dem Winkeleinstellglied direkt oder indirekt zusammenwirkend angeordnet oder ausgebildet ist derart, dass das Schwenkelement um die Schwenkachse verschwenkbar ist durch Verdrehen des Winkeleinstellglieds um die Winkeleinstellglieddrehachse. Insbesondere kann so ein besonders kompakter Aufbau der Ausrichtvorrichtung realisiert werden, wenn beispielsweise das Winkeleinstellglied und das Schwenkelement direkt zusammenwirken. So kann auf ein komplexes Umlenkgetriebe verzichtet werden.

Besonders einfach lassen sich Kräfte zum Verschwenken des Schwenkelements übertragen, wenn die Schwenkachse parallel zur Winkeleinstellglieddrehachse verläuft.

Vorzugsweise ist das erste Kopplungsglied am Schwenkelement angeordnet oder ausgebildet. Insbesondere kann das erste Kopplungsglied einstückig mit dem Schwenkelement ausgebildet sein. Diese Anordnung ermöglicht es insbesondere, die Ausrichtvorrichtung derart auszubilden, dass eine mit der Winkeländerungseinrichtung eingestellte Ausrichtung des ersten Kopplungsglieds erhalten bleibt, wenn mit der Abstandsänderungseinrichtung das Schiebeelement relativ zum Grundkörper verschoben wird.

Günstig ist es, wenn das erste Kopplungsglied eine Kopplungsgliedlängsachse definiert und wenn die Kopplungsgliedlängsachse quer zur Schiebeelementlängsachse verläuft. So lässt sich insbesondere eine Höhenverstellung von einer Neigungsverstellung auf einfache Weise trennen.

Eine Kopplung der Ausrichtvorrichtung mit einer Sägeschablone wird insbesondere dann besonders einfach möglich, wenn die Kopplungsgliedlängsachse quer, insbesondere senkrecht, zur Schwenkachse verläuft.

Günstig ist es, wenn der Grundkörper zwei Lagerbacken umfasst, wenn das Schwenkelement zwischen den Lagerbacken auf einem an den Lagerbacken gehaltenen Lagerstift gelagert ist oder wenn die Lagerbacken auf zwei am Schwenkelement abstehenden Lagervorsprüngen gelagert sind. Auf diese Weise lässt sich das Schwenkelement einfach und kompakt am Grundkörper verschwenkbar lagern.

Eine besonders kompakte Ausbildung der Ausrichtvorrichtung kann insbesondere dadurch erreicht werden, dass der Lagerstift oder die Lagervorsprünge die Schwenkachse definieren.

Ferner ist es günstig, wenn die Winkeländerungseinrichtung einen exzentrisch zur Winkeleinstellglieddrehachse angeordneten Schwenkmitnehmer umfasst, wenn der Schwenkmitnehmer am Winkeleinstellglied angeordnet oder ausgebildet ist und mit dem Schwenkelement beweglich in Eingriff steht oder wenn der Schwenkmitnehmer am Schwenkelement angeordnet oder ausgebildet ist und mit dem Winkeleinstellglied beweglich in Eingriff steht. Diese Ausgestaltung ermöglicht es insbesondere, eine Betätigungskraft, die über das Winkeleinstellglied eingeleitet wird, über den Schwenkmitnehmer direkt auf das Schwenkelement zu übertragen, um eine Ausrichtung des ersten Kopplungsglieds zu ändern, insbesondere den Winkel zwischen der Befestigungselementaufnahme und dem ersten Kopplungsglied. Exzentrisch bedeutet insbesondere, dass eine Position des Schwenkmitnehmers von der Winkeleinstellglieddrehachse beabstandet ist.

Besonders einfach und kostengünstig ausbilden lässt sich die Ausrichtvorrichtung, wenn der Schwenkmitnehmer in Form eines Stifts ausgebildet ist und wenn eine Stiftlängsachse des Stifts parallel oder im Wesentlichen parallel zur Winkeleinstellglieddrehachse verläuft. Insbesondere kann die Stiftlängsachse parallel zur Schwenkachse verlaufen.

Eine Kraftübertragung zwischen dem Winkeleinstellglied und dem Schwenkelement lässt sich auf besonders einfache Weise erreichen, wenn der Schwenkmitnehmer in eine korrespondierende Schwenkmitnehmerführungsausnehmung eingreift zum Übertragen einer Antriebskraft vom Winkeleinstellglied auf das Schwenkelement.

Vorzugsweise verläuft die Schwenkachse parallel oder im Wesentlichen parallel zur Abstandseinstellglieddrehachse. Dies ermöglicht einen besonders kompakten Aufbau der Ausrichtvorrichtung.

Um eine Winkelverstellung mit besonders geringen Kräften vornehmen zu können, ist es günstig, wenn ein Abstand der Schwenkachse von der Winkeleinstellglieddrehachse größer ist als ein Abstand des Schwenkmitnehmers von der Winkeleinstellglieddrehachse.

Vorteilhaft ist es, wenn ein Abstand des Schiebemitnehmers von der Abstandseinstellglieddrehachse größer ist als ein Abstand des Schwenkmitnehmers von der Abstandseinstellglieddrehachse.

Vorzugsweise ist ein Abstand der Schwenkachse von der Winkeleinstellglieddrehachse größer als ein Abstand der Schwenkachse vom Schwenkmitnehmer. So kann das Schwenkelement mit besonders kleinen Schwenkkräften verschwenkt werden.

Günstig ist es, wenn die Abstandsänderungseinrichtung ausgebildet ist zum Verändern des Abstands zwischen der Befestigungselementaufnahme und dem ersten Kopplungsglied in diskreten Abstandsschritten. Beispielsweise kann die Abstandsänderungseinrichtung so auch derart ausgebildet werden, dass sie ihre eingestellte Position beibehält. Zudem kann ein Operateur insbesondere auch eine Rückmeldung über eine Anzahl von ihm vorgenommener Abstandsänderungsschritte zum Ändern des Abstands erhalten.

Um die Abstände in definierter Weise von einem Operateur einstellen zu können, ist es vorteilhaft, wenn die diskreten Abstandsschritte gleich groß oder im Wesentlich gleich groß sind.

Vorzugsweise umfasst die Abstandsänderungseinrichtung eine Abstandsschritteinstelleinrichtung zum Einstellen diskreter Abstände zwischen der Befestigungselementaufnahme und dem ersten Kopplungsglied. Mit der Abstandsschritteinstelleinrichtung lassen sich diskrete Abstandsschritte vorgeben.

Vorteilhaft ist es, wenn die Abstandsschritteinstelleinrichtung in Form eines Abstandsrastmechanismus ausgebildet ist umfassend miteinander zusammenwirkende erste und zweite Abstandsrastglieder und wenn die ersten und zweiten Abstandsrastglieder bei diskreten Abständen zwischen der Befestigungselementaufnahme und dem ersten Kopplungsglied in Eingriff stehen. Ein solcher Abstandsrastmechanismus ermöglicht es insbesondere, einem Operateur eine haptische Rückmeldung bei einer Änderung eines Abstands zu geben.

Auf einfache Weise lässt sich die Abstandsschritteinstelleinrichtung ausbilden, wenn die ersten und zweiten Abstandsrastglieder einerseits am Abstandseinstellglied und andererseits am Grundkörper angeordnet oder ausgebildet sind.

Vorzugsweise ist eine Mehrzahl erster Abstandsrastglieder in Form von eine Abstandsverzahnung ausbildenden Zähne ausgebildet. Die Abstandsverzahnung kann beispielsweise in Form einer Linearverzahnung oder einer Bogenverzahnung ausgebildet sein.

Eine besonders kompakte Ausbildung der Ausrichtvorrichtung kann erreicht werden, wenn die Abstandsverzahnung die Abstandseinstellglieddrehachse konzentrisch umgebend angeordnet oder ausgebildet ist. So kann die Abstandsverzahnung beispielsweise mit einem zweiten, in einem gleichbleibenden Abstand zur Abstandseinstellglieddrehachse angeordneten Rastglied sicher zusammenwirken.

Vorteilhaft ist es, wenn ein zweites Abstandsrastglied in Form eines gegen ein vorspannendes Element aus einer Grundstellung auslenkbaren Abstandsrastkörpers ausgebildet ist. Der Abstandsrastkörper kann so entgegen der Wirkung des vorspannenden Elements bewegt werden und bei Verdrehen des Abstandseinstellglieds sukzessive zwischen benachbarte Zähne der Abstandsverzahnung eingreifen.

Ein besonders kompakter Aufbau der Ausrichtvorrichtung wird insbesondere dadurch ermöglicht, dass eine Wirkrichtung des vorspannenden Elements parallel oder im Wesentlichen parallel zur Abstandseinstellglieddrehachse verläuft.

Vorteilhaft ist es, wenn die ersten Abstandsrastglieder in Richtung auf den Grundkörper hin weisend vom Abstandseinstellglied abstehend angeordnet oder ausgebildet sind. Insbesondere kann das Abstandseinstellglied die Verzahnung umfassen oder tragen.

Um einen eingestellten Abstand einfach und sicher halten zu können, auch unter Belastung beim Einsatz einer Sägeschablone, ist es günstig, wenn ein Abstand der ersten Abstandsrastglieder von der Abstandseinstellglieddrehachse größer ist als ein Abstand des Schiebemitnehmers von der Abstandseinstellglieddrehachse.

Gemäß einer weiteren bevorzugten Ausführungsform der Erfindung kann vorgesehen sein, dass die Winkeländerungseinrichtung eine Winkelschritteinstelleinrichtung umfasst zum Einstellen diskreter Winkel zwischen der Befestigungselementaufnahme und dem ersten Kopplungsglied. Dies ermöglicht es auf einfache Weise, den Winkel in diskreten Schritten, beispielsweise in Schritten von 1°, durch einen Operateur zu ändern, um so eine optimale und präzise Einstellung und Positionierung der Sägeschablone zu erhalten.

Günstig ist es, wenn die Winkelschritteinstelleinrichtung in Form eines Winkelrastmechanismus ausgebildet ist umfassend miteinander zusammenwirkende erste und zweite Winkelrastglieder und wenn die ersten und zweiten Winkelrastglieder bei diskreten Winkeln zwischen der Befestigungselementaufnahme und dem ersten Kopplungsglied in Eingriff stehen. So lassen sich auf einfache Weise diskrete Winkelschritte durch einen Operateur einstellen.

Einfach und kompakt ausbilden lässt sich die Ausrichtvorrichtung, wenn die ersten und zweiten Winkelrastglieder einerseits am Winkeleinstellglied und andererseits am Grundkörper angeordnet oder ausgebildet sind.

Um eine Mehrzahl diskreter Winkelschritte vorgeben zu können, ist es günstig, wenn eine Mehrzahl erster Winkelrastglieder in Form von eine Winkelverzahnung ausbildenden Zähnen ausgebildet ist. Eine solche Winkelverzahnung kann insbesondere mit einem einzigen zweiten Winkelrastglied zusammenwirken, welches zwischen benachbarte Zähne der Winkelverzahnung eingreifen und so definierte Winkelstellungen vorgeben kann.

Vorzugsweise ist die Winkelverzahnung die Winkeleinstellglieddrehachse konzentrisch umgebend angeordnet oder ausgebildet. Dies ermöglicht einen einfachen und kompakten Aufbau der Winkeländerungseinrichtung.

Günstig ist es, wenn ein zweites Winkelrastglied in Form eines gegen ein vorspannendes Element aus einer Grundstellung auslenkbaren Winkelrastkörpers ausgebildet ist. So lässt sich insbesondere ein Kugeldruckstück ausbilden, wenn der Winkelrastkörper in Form einer Kugel und das vorspannende Element in Form einer Druckfeder ausgebildet sind.

Ein besonders kompakter Aufbau der Ausrichtvorrichtung kann insbesondere dadurch erreicht werden, dass dem zweiten Abstandsrastglied und dem zweiten Winkelrastglied ein gemeinsames vorspannendes Element zugeordnet ist. So kann praktisch ein Doppelkugeldruckstück ausgebildet werden, welches einerseits mit der Winkelverzahnung und andererseits mit der Abstandsverzahnung zusammenwirkt. Das vorspannende Element kann insbesondere in einer Ausnehmung, beispielsweise einer Bohrung, des Grundkörpers angeordnet sein.

Vorzugsweise verläuft eine Wirkrichtung des vorspannenden Elements parallel oder im Wesentlichen parallel zur Winkeleinstellglieddrehachse. So können maximale Rastkräfte beim Zusammenwirken der ersten und zweiten Winkelrastglieder vorgegeben werden. Entsprechend gilt dies auch für das vorspannende Element, welches mit dem zweiten Abstandsrastglied zusammenwirkt.

Vorteilhaft ist es, wenn die ersten Winkelrastglieder in Richtung auf den Grundkörper hin weisend vom Winkeleinstellglied abstehend angeordnet oder ausgebildet sind. So können sie auf einfache Weise mit einem aus dem Grundkörper vorstehenden zweiten Winkelrastglied zusammenwirken.

Günstig ist es, wenn ein Abstand der ersten Winkelrastglieder von der Winkeleinstellglieddrehachse größer ist als ein Abstand des Schwenkmitnehmers von der Winkeleinstellglieddrehachse. So kann insbesondere ein eingestellter Winkel einfach und sicher gehalten werden, auch unter Belastung beim Einsatz einer Sägeschablone.

Ferner ist es vorteilhaft, wenn Befestigungselementaufnahme am Schiebeelement angeordnet oder ausgebildet ist. Insbesondere kann die Befestigungselementaufnahme in Form einer im Querschnitt rotationssymmetrischen Durchbrechung ausgebildet sein, beispielsweise in Form einer Bohrung. Eine solche Ausgestaltung ermöglicht es insbesondere, das Schiebeelement und damit die gesamte Ausrichtvorrichtung um eine von der Befestigungselementaufnahme definierte Dreh- oder Schwenkachse zu verschwenken. So kann zunächst eine Grobpositionierung der Sägeschablone am Knochen vorgenommen werden. Eine Feinjustierung erfolgt dann mit der Abstandsänderungseinrichtung und mit der Winkeländerungseinrichtung. Zudem ermöglicht es diese Anordnung der Befestigungselementaufnahme, die Änderung des Abstands und des Winkels quasi in Serie geschaltet vorzunehmen. Der Grundkörper kann relativ zum Schiebeelement und damit relativ zur Befestigungselementaufnahme durch die Abstandsänderungseinrichtung verschoben werden. Ferner kann das erste Kopplungsglied relativ zum Grundkörper und damit auch zur Befestigungselementaufnahme verschwenkt werden, unabhängig von einer Abstandsänderung. Ferner kann die Befestigungselementaufnahme in Form einer langgestreckten Hülse ausgebildet sein, um eine Stabilität einer Verbindung zwischen dem Schiebeelement und dem Befestigungselement zu verbessern. Insbesondere kann so ein Spiel zwischen dem die Befestigungselementaufnahme durchsetzenden Befestigungselement und dem Schiebeelement minimiert werden.

Günstig ist es, wenn das medizinische Sägeschablonensystem mindestens ein Befestigungselement zum Verankern in einem Knochen umfasst. Ein Befestigungselement kann insbesondere dazu dienen, die Ausrichtvorrichtung am Knochen anzuordnen und zu positionieren. Dieses Befestigungselement wird dann durch die Befestigungselementaufnahme der Ausrichtvorrichtung geführt, die insbesondere am Schiebeelement angeordnet sein kann. Weitere Befestigungselemente können insbesondere dazu dienen, um die von einem Operateur in eine gewünschte Position gebrachte Sägeschablone am Knochen zu fixieren, bevor er mit einer chirurgischen Säge einen durch die Sägeschablone geführten Sägeschnitt am Knochen setzt.

Auf einfache Weise lässt sich das Sägeschablonensystem an einem Knochen fixieren, wenn das mindestens eine Befestigungselement in Form eines Knochenpins oder in Form einer Knochenschraube ausgebildet ist. Insbesondere kann das Befestigungselement zylindrische Abschnitte aufweisen, die in der Befestigungselementaufnahme der Ausrichtvorrichtung verdrehbar geführt werden können.

Günstig ist es, wenn das Sägeschablonensystem eine Sägeschablone mit mindestens einer Führungsdurchbrechung für ein Sägeblatt umfasst. Die Führungsdurchbrechung kann insbesondere in Form eines Führungsschlitzes ausgebildet sein. Die Sägeschablone kann auch zwei, drei oder mehr Führungsschlitze aufweisen.

Um die Sägeschablone an einem Knochen in definierter Weise festlegen zu können, ist es günstig, wenn die Sägeschablone mindestens eine Sägeschablonenbefestigungselementaufnahme zum Aufnehmen mindestens eines Befestigungselements umfasst. Vorzugsweise sind mindestens zwei Sägeschablonenbefestigungselementaufnahmen vorgesehen, die Längsachsen definieren, welche nicht parallel zueinander verlaufen.

Vorteilhaft kann es ferner sein, wenn die Sägeschablone ein zweites Kopplungsglied umfasst zum in Eingriff Bringen mit dem ersten Kopplungsglied. So kann die Sägeschablone bei Bedarf von der Ausrichtvorrichtung getrennt werden. Insbesondere lässt sich so auch die Ausrichtvorrichtung von der Sägeschablone lösen, wenn die Sägeschablone in einer definierten Position am Knochen mit entsprechenden Befestigungselementen fixiert ist. Die dann störende Ausrichtvorrichtung kann vor dem Setzen eines Sägeschnitts entfernt werden, um den Operationssitus so gut wie möglich frei zu geben.

Ferner ist es günstig, wenn die Sägeschablone mit dem ersten Kopplungsglied unlösbar verbunden oder mit dem ersten Kopplungsglied einstückig ausgebildet ist. So lässt sich eine besonders spielfreie Verbindung zwischen der Befestigungselementaufnahme der Ausrichtvorrichtung und der Sägeschablone ausbilden.

Um eine Position der Sägeschablone im Raum möglichst genau bestimmen zu können, ist es vorteilhaft, wenn die Sägeschablone mindestens ein erstes Kopplungselement umfasst zum in Eingriff Bringen mit einem zweiten Kopplungselement einer medizinischen Referenzierungseinheit. Die medizinische Referenzierungseinheit kann insbesondere ausgebildet sein, um von einem medizinischen Navigationssystem, insbesondere einer Nachweiseinrichtung desselben, detektiert zu werden, um die Position und/oder Orientierung der Sägeschablone im Raum feststellen zu können. Insbesondere kann so eine Ausrichtung der Sägeschablone am Knochen navigationsgestützt von einem Operateur vorgenommen werden.

Vorteilhaft ist es, wenn das Sägeschablonensystem eine medizinische Referenzierungseinheit umfasst, deren Position und/oder Orientierung im Raum mit einem medizinischen Navigationssystem bestimmbar ist. Wie beschrieben kann so eine Sägeschablone navigationsgestützt am Knochen positioniert und relativ zu diesem ausgerichtet werden, um die für die Implantation von Gelenkimplantaten erforderlichen Knochenflächen zu präparieren.

Gemäß einer weiteren bevorzugten Ausführungsform der Erfindung kann vorgesehen sein, dass die Referenzierungseinheit an der Sägeschablone angeordnet oder ausgebildet ist oder dass die Referenzierungseinheit ein zweites Kopplungselement umfasst zum in Eingriff Bringen mit dem ersten Kopplungselement der Sageschablone in einer Kopplungsstellung. Insbesondere kann so die Referenzierungseinheit von der Sägeschablone abgenommen werden, wenn diese in gewünschter Weise positioniert und am Knochen fixiert ist. So hat ein Operateur mehr Platz am Operationssitus zur Verfügung.

Die nachfolgende Beschreibung bevorzugter Ausführungsformen der Erfindung dient im Zusammenhang mit den Zeichnungen der näheren Erläuterung. Es zeigen:
- Figur 1:: eine schematische perspektivische Gesamtansicht eines medizinischen Sägeschablonensystems, das an einer Tibia fixiert ist;

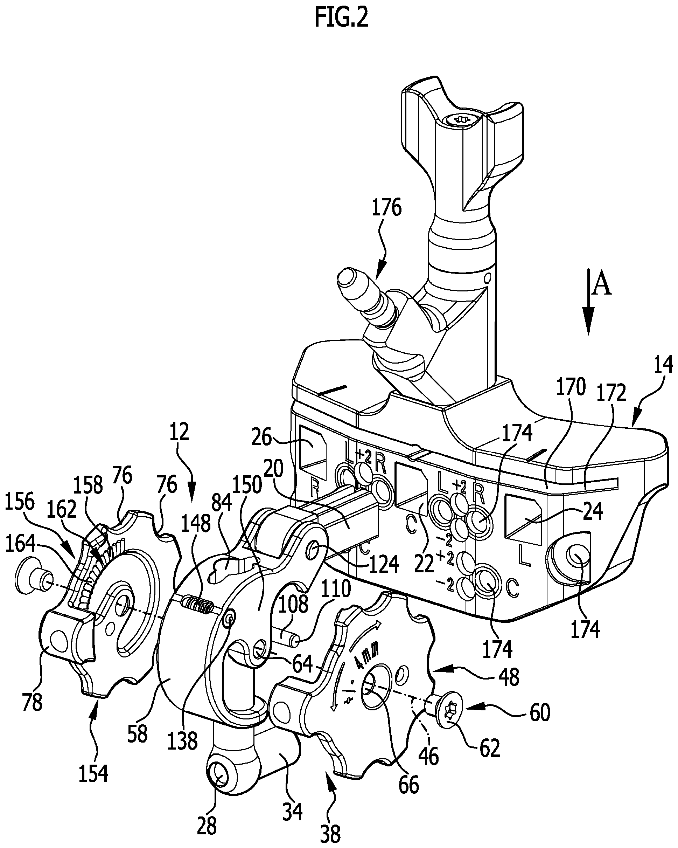

- Figur 2:: eine teilweise perspektivische Explosionsdarstellung des Sägeschablonensystems aus Figur 1;

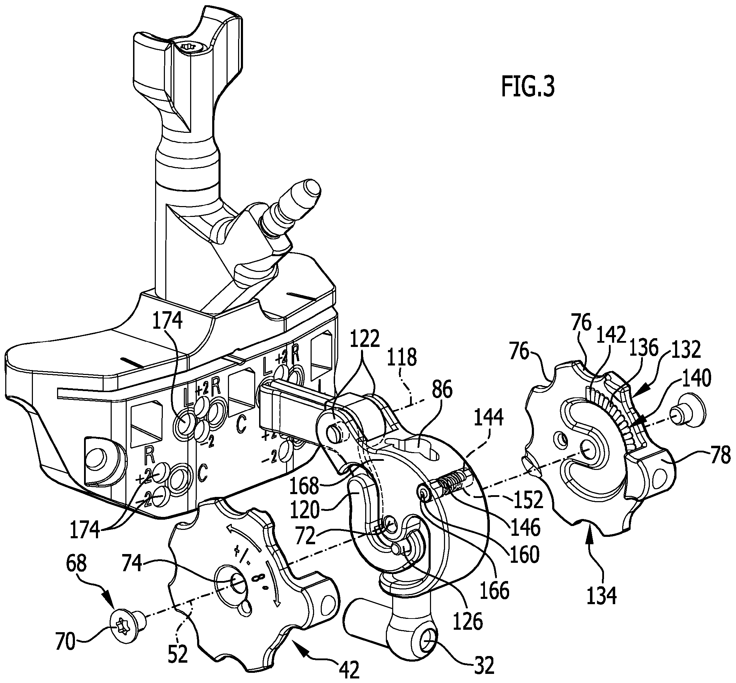

- Figur 3:: eine weitere perspektivische Ansicht der Anordnung aus Figur 2;

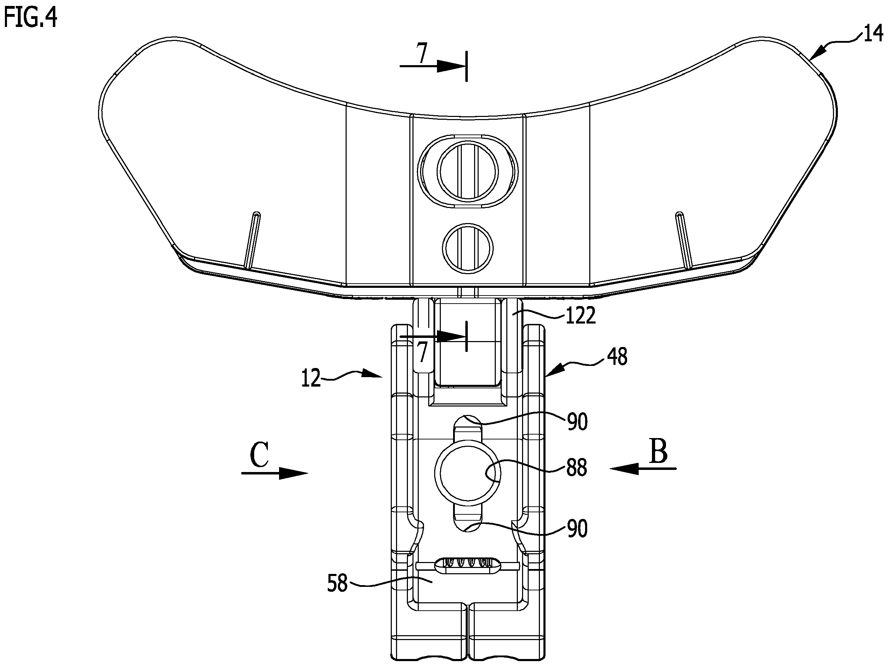

- Figur 4:: eine Ansicht der Anordnung aus Figur 2 in Richtung des Pfeils A;

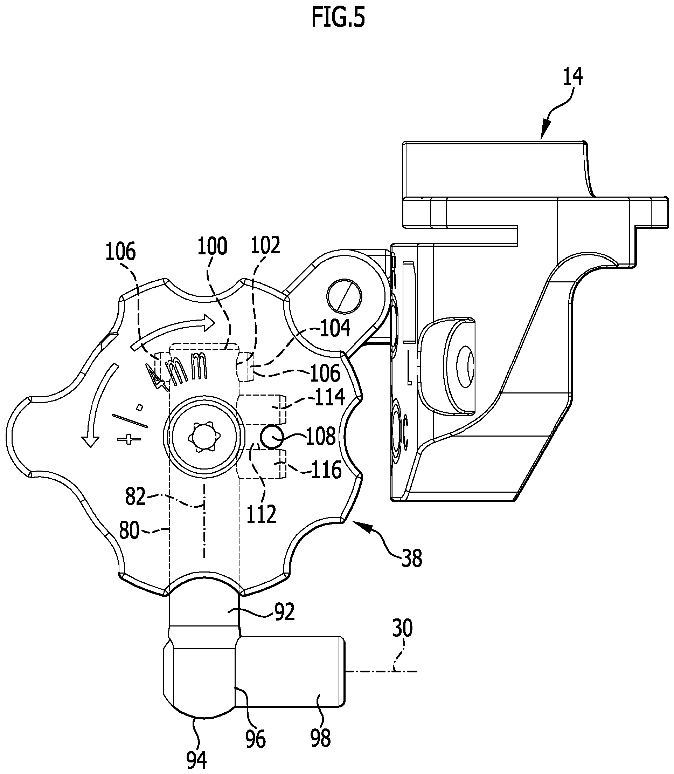

- Figur 5:: eine Ansicht der Anordnung aus Figur 4 in Richtung des Pfeils B;

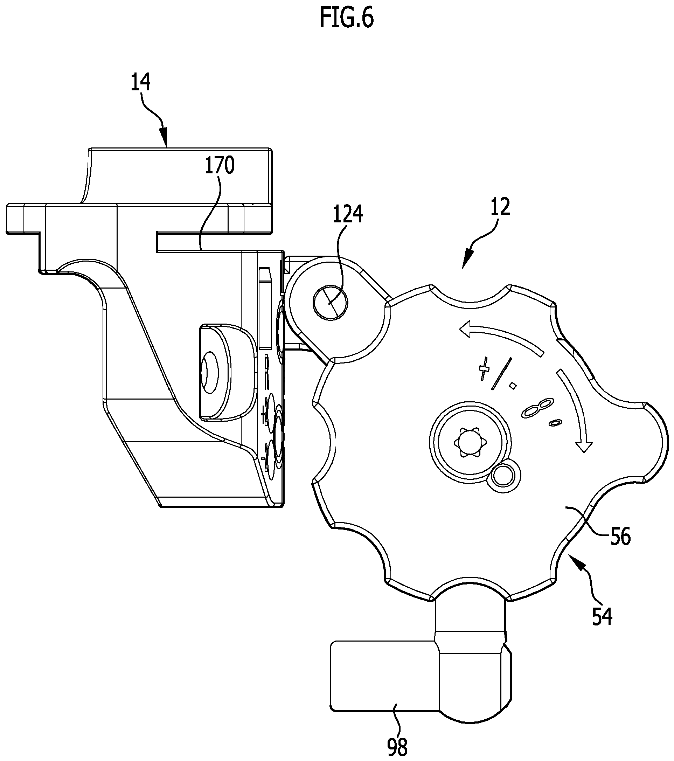

- Figur 6:: eine Ansicht der Anordnung aus Figur 4 in Richtung des Pfeils C; und

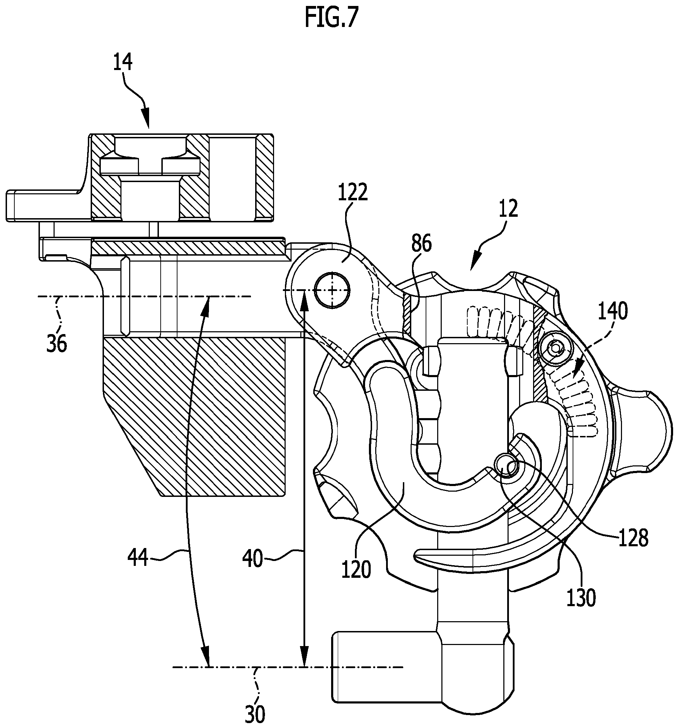

- Figur 7:: eine Schnittansicht längs Linie 7-7 in Figur 4.

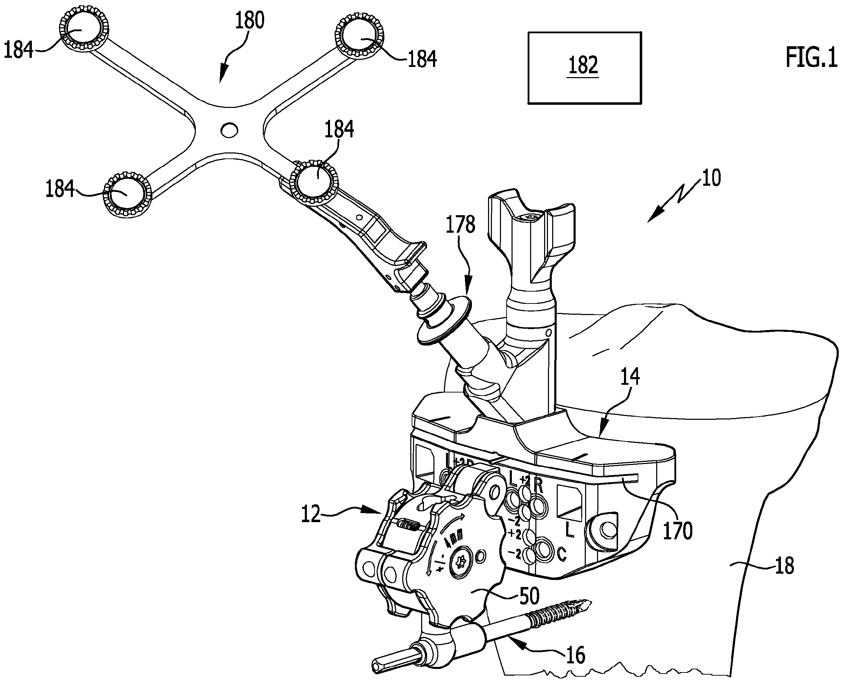

In Figur 1 ist schematisch ein insgesamt mit dem Bezugszeichen 10 bezeichnetes medizinisches Sägeschablonensystem dargestellt. Es umfasst eine Ausrichtvorrichtung 12 sowie optional eine Sägeschablone 14 sowie optional ein oder mehrere Befestigungselemente 16 zum Festlegen an einem Knochen 18.

Das Befestigungselement 16 kann insbesondere in Form eines Knochenpins oder einer Knochenschraube ausgebildet sein.

Die Ausrichtvorrichtung 12 umfasst ein erstes, im Wesentlichen quaderförmiges Kopplungsglied 20, welches eine Schnittstelle zum temporären Koppeln mit der Sägeschablone 14 bildet. Das zapfenförmige Kopplungsglied 20 umfasst eine nicht dargestellte Blattfeder, um es kraft- und/oder formschlüssig mit einem an der Sägeschablone 14 ausgebildeten zweiten Kopplungsglied 22 in Eingriff zu bringen. Das zweite Kopplungsglied 22 ist in Form einer quaderförmigen Ausnehmung ausgebildet.

Optional können auch weitere zweite Kopplungsglieder 24 und 26 an der Sägeschablone 14 ausgebildet sein, um die Sägeschablone 14 in unterschiedlichen Relativpositionen mit der Ausrichtvorrichtung 12 zu koppeln.

Die Ausrichtvorrichtung 12 umfasst ferner eine Befestigungselementaufnahme 28. Diese ist in Form einer eine Längsachse 30 definierenden Durchbrechung in Form einer Bohrung 32 einer Führungshülse 34 ausgebildet.

Das erste Kopplungsglied 20 definiert eine Kopplungsgliedlängsachse 36.

Ferner umfasst die Ausrichtvorrichtung 12 eine Abstandsänderungseinrichtung 38 zum Verändern eines Abstands 40 zwischen der Befestigungselementaufnahme 28 und dem ersten Kopplungsglied 20.

Ferner umfasst die Ausrichtvorrichtung 12 eine Winkeländerungseinrichtung 42 zum Verändern eines Winkels 44 zwischen der Befestigungselementaufnahme 28 und dem ersten Kopplungsglied 20, und zwar insbesondere zum Einstellen des Winkels 44, welcher durch die Längsachse 30 und die Kopplungsgliedlängsachse 36 eingeschlossen wird.

Zum Ändern des Abstands 40 umfasst die Abstandsänderungseinrichtung 38 ein um eine Abstandseinstellglieddrehachse 46 verdrehbares Abstandseinstellglied 48, welches in Form eines Einstellrads 50 ausgebildet ist.

Die Winkeländerungseinrichtung 42 umfasst ein um eine Winkeleinstellglieddrehachse 52 verdrehbares Winkeleinstellglied 54, welches ebenfalls in Form eines Einstellrads 56 ausgebildet ist.

Bei der in den Figuren dargestellten Ausrichtvorrichtung 12 fallen die Abstandseinstellglieddrehachse 46 und die Winkeleinstellglieddrehachse 52 zusammen. Mit anderen Worten definiert die Abstandseinstellglieddrehachse 46 die Winkeleinstellglieddrehachse 52.

Die Abstandsänderungseinrichtung 38 ist unabhängig von der Winkeländerungseinrichtung 42 betätigbar.

Die Ausrichtvorrichtung 12 umfasst einen im Wesentlichen halbmondförmigen Grundkörper, an dem sowohl das Abstandseinstellglied 48 als auch das Winkeleinstellglied 54 verdrehbar gelagert beziehungsweise gehalten sind.

Dem Abstandseinstellglied 48 ist ein Abstandseinstellgliedlagerelement 60 in Form einer Schraube 62 zugeordnet, deren Schaft mit einem Außengewinde versehen ist, das zu einem Innengewinde einer Gewindebohrung 64 am Grundkörper 58 korrespondiert. Der Schaft der Schraube 62 durchsetzt eine Durchbrechung 66 am Einstellrad 50, sodass das Einstellrad 50 durch Einschrauben des Schafts der Schraube 62 in die Gewindebohrung 64 am Grundkörper 58 verdrehbar befestigt werden kann.

In analoger Weise ist dem Winkeleinstellglied 54 ein Winkeleinstellgliedlagerelement 68 in Form einer Schraube 70 zugeordnet, deren mit einem Außengewinde versehener Schaft mit einer Gewindebohrung 72 am Grundkörper 58 verschraubt werden kann. Das Einstellrad 56 ist mit einer Durchbrechung 74 versehen, sodass es entsprechend dem Einstellrad 50 verdrehbar mit dem Grundkörper 58 verbunden werden kann.

Sowohl das Abstandseinstellglied 48 als auch das Winkeleinstellglied 54 weisen mehrere in radialer Richtung von der Abstandseinstellglieddrehachse 46 weg weisende Ausnehmungen 76 auf, sodass eine Art Außenverzahnung der Einstellräder 50 und 56 ausgebildet wird.

Ferner ist auch an jedem Einstellrad 50, 56 ein Vorsprung 78 ausgebildet, welcher in radialer Richtung von der Abstandseinstellglieddrehachse 46 weg weisend vorsteht. Die beiden Vorsprünge 78 erstrecken sich zudem parallel zur Abstandseinstellglieddrehachse 46, weisen jeweils aufeinander zu und weisen zudem jeweils eine senkrecht zur Abstandseinstellglieddrehachse 46 verlaufende Bohrung auf, welche beispielsweise mit einem Hebel temporär koppelbar ist.

Die Abstandsänderungseinrichtung 38 umfasst ferner ein Schiebeelement 80, welches eine Schiebeelementlängsachse 82 definiert. Die Schiebeelementlängsachse 82 verläuft senkrecht zur Längsachse 30.

Am Grundkörper 58 ist eine Schiebeelementführung 84 ausgebildet in Form einer Durchbrechung 86, welche einen unrunden Querschnitt aufweist. Die Durchbrechung 86 ist gebildet durch eine die Schiebeelementlängsachse 82 definierende Bohrung 88 sowie zwei seitlich in radialer Richtung von der Schiebeelementlängsachse 82 weg weisend ausgebildete, einander diametral bezogen auf die Schiebeelementlängsachse 82 gegenüberliegende Aussparungen 90.

Das Schiebeelement 80 ist in Form eines zylindrischen Bolzens 92 ausgebildet, welcher benachbart einem freien Ende 94 mit einer Bohrung 96 versehen ist, in die eine Hülse 98 eingesetzt ist, welche mit der Bohrung 32 zur Ausbildung der Befestigungselementaufnahme 28 versehen ist.

Einem zweiten, vom Ende 94 weg weisenden Ende 100 benachbart ist eine weitere Bohrung 102 ausgebildet, in die ein Führungsstift 104 eingesetzt ist, dessen voneinander weg weisende freie Ende 106 seitlich über den Bolzen 92 vorragen. Eine Längsachse des Führungsstifts 104 ist parallel zur Längsachse 30 ausgerichtet. Die Enden 106 greifen in die seitlichen Aussparungen 90 ein, sodass das Schiebeelement 80 in der Schiebeelementführung parallel zur Schiebeelementlängsachse 82 verschiebbar und gegen eine Verdrehung um diese gesichert ist.

Die Abstandsänderungseinrichtung 38 umfasst ferner einen Schiebemitnehmer 108, welcher am Abstandseinstellglied 48 bezogen auf die Abstandseinstellglieddrehachse 46 exzentrisch angeordnet ist. Der Schiebemitnehmer 108 ist in Form eines Stifts 110 ausgebildet, dessen Stiftlängsachse parallel zur Abstandseinstellglieddrehachse 46 verläuft.

Der Schiebemitnehmer 108 greift in eine Führungsausnehmung 112 ein. Diese ist ausgebildet durch zwei Stifte 114 und 116, die voneinander einen Abstand aufweisen, welcher einem Durchmesser des Stifts 110 entspricht.

Die Stifte 114 und 116 sind in Bohrungen oder Sacklöcher am Schiebeelement 80 eingesetzt und derart ausgerichtet, dass ihre Längsachsen parallel zur Längsachse 30 verlaufen. Auf diese Weise steht das Schiebeelement 80 beweglich mit dem Schiebemitnehmer 108 in Eingriff, sodass eine Antriebskraft vom Abstandseinstellglied 48 auf das Schiebeelement 80 zum Bewegen desselben nach dem Exzenterprinzip übertragen werden kann. Durch Verdrehen des Einstellrads 50 wird der Schiebemitnehmer 108 um die Abstandseinstellglieddrehachse 46 verdreht, sodass er das Schiebeelement 80 in der Schiebeelementführung 84 verschieben kann.

Die Schiebeelementlängsachse 82 verläuft senkrecht zur Abstandseinstellglieddrehachse 46.

In der beschriebenen Weise ist das Abstandseinstellglied 48 direkt mit dem Schiebeelement 80 zusammenwirkend ausgebildet.

Die Winkeländerungseinrichtung 42 umfasst ein am Grundkörper 58 um eine Schwenkachse 118 verschwenkbar gelagertes Schwenkelement 120, welches in Form eines Schwenkhebels ausgebildet ist.

Die Schwenkachse 118 verläuft parallel zur Winkeleinstellglieddrehachse 52.

Das erste Kopplungsglied 20 ist am Schwenkelement 120 ausgebildet und bildet ein freies Ende desselben. Die Kopplungsgliedlängsachse 36 verläuft senkrecht zur Schwenkachse 118, und zwar unabhängig davon, wie das Schwenkelement 120 relativ zum Grundkörper 58 um die Schwenkachse 118 verschwenkt ist.

Am Grundkörper 58 stehen parallel zueinander zwei flache Lagerbacken 122 ab, zwischen denen das Schwenkelement 120 auf einem an den Lagerbacken 122 gehaltenen Lagerstift 124 gelagert ist. Der Lagerstift 124 definiert die Schwenkachse 118.

Die Winkeländerungseinrichtung 42 umfasst einen Schwenkmitnehmer 126, welcher bezogen auf die Winkeleinstellglieddrehachse 52 exzentrisch am Winkeleinstellglied 24 angeordnet ist. Der Schwenkmitnehmer 126 ist in Form eines Stifts 128 ausgebildet, dessen Stiftlängsachse parallel zur Winkeleinstellglieddrehachse 52 verläuft.

Der Schwenkmitnehmer 126 steht mit dem Schwenkelement 120 beweglich in Eingriff, und zwar indem der Schwenkmitnehmer 126 in eine korrespondierende Schwenkmitnehmerführungsausnehmung 130 eingreift zum Übertragen einer Antriebskraft vom Winkeleinstellglied auf das Schwenkelement 120 nach dem Exzenterprinzip.

Die Schwenkachse 118 verläuft zudem parallel zur Abstandseinstellglieddrehachse 46 und damit auch parallel zur Winkeleinstellglieddrehachse 52.

Ein Abstand der Schwenkachse 118 von der Winkeleinstellglieddrehachse 52 ist größer als ein Abstand des Schwenkmitnehmers 108 beziehungsweise dessen Längsachse von der Winkeleinstellglieddrehachse 52.

Ferner ist ein Abstand des Schiebemitnehmers 108 beziehungsweise dessen Längsachse von der Abstandseinstellglieddrehachse 46 größer als ein Abstand des Schwenkmitnehmers 108 von der Abstandseinstellglieddrehachse 46.

Außerdem ist ein Abstand der Schwenkachse 118 von der Winkeleinstellglieddrehachse 52 größer als ein Abstand der Schwenkachse 118 vom Schwenkmitnehmer 108 beziehungsweise dessen Längsachse.

Wird das Einstellrad 56 um die Winkeleinstellglieddrehachse 52 verdreht, nimmt der Schwenkmitnehmer 126 den Schwenkhebel mit und verschwenkt diesen um die Schwenkachse 118. Dadurch ändert sich der Winkel 44 zwischen der Kopplungsgliedlängsachse 36 und der Längsachse 30. Mit anderen Worten kann eine Neigung des ersten Kopplungsglieds 20 relativ zur Längsachse 30 beziehungsweise zur Befestigungselementaufnahme 28 durch Drehen beziehungsweise Verdrehen des Winkeleinstellglieds 54 eingestellt beziehungsweise geändert werden.

Ferner ist die Ausrichtvorrichtung 12 derart ausgebildet, dass der Abstand 40 mit der Abstandsänderungseinrichtung in diskreten Abstandsschritten verändert werden kann. Diese können insbesondere gleich groß oder im Wesentlichen gleich groß sein. Beispielsweise können die Abstandsschritte 0,5 mm, 1 mm oder 1,5 mm betragen.

Die Abstandsänderungseinrichtung 38 umfasst eine Abstandsschritteinstelleinrichtung 132 zum Einstellen diskreter Abstände 40 zwischen der Befestigungselementaufnahme 28 und dem ersten Kopplungsglied 20.

Die Abstandsschritteinstelleinrichtung 132 ist in Form eines Abstandsrastmechanismus 134 ausgebildet und umfasst miteinander zusammenwirkende erste Abstandsrastglieder 136 und ein zweites Abstandsrastglied 138. Die ersten und zweiten Abstandsrastglieder 136, 138 stehen bei diskreten Abständen 40 in Eingriff.

Der Abstandsrastmechanismus 134 umfasst eine Mehrzahl erster Abstandsrastglieder 136, welche in Form von eine Abstandsverzahnung 140 ausbildenden Zähnen 142 ausgebildet sind. Die ersten Abstandsrastglieder 136 sind am Abstandseinstellglied 48 ausgebildet beziehungsweise angeordnet, das zweite Abstandsrastglied ist am Grundkörper 58 angeordnet oder ausgebildet.

Die Abstandsverzahnung 140 ist die Abstandseinstellglieddrehachse 46 konzentrisch umgebend angeordnet oder ausgebildet und erstreckt sich etwa über einen Winkelbereich von 90° bezogen auf die Abstandseinstellglieddrehachse 46.

Das zweite Abstandsrastglied 138 ist in Form eines kugelförmigen Abstandsrastkörpers 144 ausgebildet, welcher entgegen der Wirkung eines vorspannenden Elements 146, welches in Form einer Druckfeder 148 ausgebildet ist, aus einer Grundstellung, in der es über eine in Richtung auf das Einstellrad 50 hin weisende Seitenfläche 150 des Grundkörpers 58 vorsteht, ausgelenkt werden kann.

Das vorspannende Element 146 ist in eine Querbohrung 152 des Grundkörpers 58 eingesetzt. Eine Längsachse der Querbohrung 152 verläuft parallel zur Abstandseinstellglieddrehachse 46. Damit verläuft auch die Wirkrichtung des vorspannenden Elements 146 parallel zur Abstandseinstellglieddrehachse 46.

Die Zähne 142 sind in Richtung auf den Grundkörper 58 hin weisend vom Abstandseinstellglied 48 abstehend angeordnet beziehungsweise ausgebildet.

Ein Abstand der ersten Abstandsrastglieder 136 von der Abstandseinstellglieddrehachse 46 ist größer als ein Abstand des Schiebemitnehmers 108 von der Abstandseinstellglieddrehachse 46.

Der Winkel 44 kann mit der Winkeländerungseinrichtung 42 ebenfalls in diskreten Winkelschritten verstellt werden. Um dies zu ermöglichen, umfasst die Winkeländerungseinrichtung 42 eine Winkelschritteinstelleinrichtung 154 zum Einstellen diskreter Winkel 44 zwischen der Befestigungselementaufnahme 28 beziehungsweise der Längsachse 30 und dem ersten Kopplungsglied 20 beziehungsweise dessen Kopplungsgliedlängsachse 36.

Die Winkelschritteinstelleinrichtung 154 ist in Form eines Winkelrastmechanismus 156 ausgebildet. Dieser umfasst miteinander zusammenwirkende erste Winkelrastglieder 158 und ein zweites Winkelrastglied 160. Bei diskreten Winkein 44 steht das zweite Winkelrastglied 160 mit zwei Winkelrastgliedern 158 in Eingriff.

Die Winkelschritteinstelleinrichtung 154 umfasst eine Mehrzahl erster Winkelrastglieder 158, welche am Einstellrad 56 in Form von eine Winkelverzahnung 162 ausbildenden Zähnen 164 ausgebildet sind. Damit sind die ersten Winkelrastglieder 158 am Winkeleinstellglied 54 angeordnet beziehungsweise ausgebildet.

Das zweite Winkelrastglied 160 ist am Grundkörper 58 angeordnet beziehungsweise ausgebildet.

Die Winkelverzahnung 162 ist die Winkeleinstellglieddrehachse 52 konzentrisch umgebend angeordnet beziehungsweise ausgebildet. Sie erstreckt sich über einen Winkelbereich von etwa 90° bezogen auf die Winkeleinstellglieddrehachse 52.

Das zweite Winkelrastglied 160 ist in Form eines kugeligen Winkelrastkörpers 166 ausgebildet. Dieser wird durch das vorspannende Element 146 etwas über eine in Richtung auf das Einstellrad 56 weisende Seitenfläche 168 des Grundkörpers vorstehend in einer Grundstellung gehalten. Der Winkelrastkörper 166 ist somit entgegen der Wirkung des vorspannenden Elements 146 aus seiner Grundstellung auslenkbar.

Die ersten Winkelrastglieder 158 sind in Richtung auf den Grundkörper 58 hin weisend vom Winkeleinstellglied 54 abstehend angeordnet beziehungsweise ausgebildet.

Ein Abstand der ersten Winkelrastglieder 158 von der Winkeleinstellglieddrehachse 52 ist größer als ein Abstand des Schwenkmitnehmers 126 von der Winkeleinstellglieddrehachse 52.

Die Sägeschablone 14 umfasst eine Führungsdurchbrechung 170 in Form eines Führungsschlitzes 172 zum Führen eines in den Figuren nicht dargestellten Sägeblatts einer chirurgischen Säge. Insbesondere kann es sich dabei um eine Oszillationssäge handeln.

Die Sägeschablone 14 kann zudem, wie beispielhaft in den Figuren dargestellt, mehrere Sägeschablonenbefestigungselementaufnahmen 174 aufweisen, durch die Befestigungselemente, beispielsweise Knochenpins oder Knochenschrauben, hindurchführbar sind. Längsachsen der Sägeschablonenbefestigungselementaufnahmen 174 können insbesondere parallel oder auch unter einem Winkel gegeneinander geneigt in Form von Bohrungen ausgebildet sein.

Die Sägeschablone 14 kann ein oder mehrere erste Kopplungselemente 176 umfassen zum in Eingriff Bringen mit einem zweiten Kopplungselement 178 einer medizinischen Referenzierungseinheit 180.

Das Sägeschablonensystem 10 kann insbesondere ein oder mehrere derartige Referenzierungseinheiten 180 umfassen. Eine Position und/oder eine Orientierung im Raum der Referenzierungseinheit 180 können insbesondere mit einem medizinischen Navigationssystem 182 bestimmt werden.

Insbesondere kann die Referenzierungseinheit 180 mehrere Markerelemente, vorzugsweise mindestens drei, umfassen, deren Position im Raum mit dem Navigationssystem 182 bestimmbar ist. Aus einer bekannten Relativpositionierung der Markerelemente 184 kann dann mit dem Navigationssystem 182 eine Position sowie eine Orientierung der Referenzierungseinheit 180 ermittelt werden. Damit sind aber auch eine Orientierung sowie eine Position der Sägeschablone 14, die mit der Referenzierungseinheit 180 gekoppelt ist, im Raum bestimmbar.

Zum Setzen eines Sägeschnitts am Knochen 18 kann insbesondere folgendermaßen vorgegangen werden.

Die Ausrichtvorrichtung 12 wird zunächst in eine Neutralstellung gebracht. In dieser sind die Einstellräder 50 und 56 so verdreht, dass sie sowohl in Richtung des Uhrzeigersinns als auch entgegen dem Uhrzeigersinn noch etwa gleich weit verdreht werden können. Die Vorsprünge 78 grenzen dann direkt aneinander an, wie beispielhaft in Figur 1 dargestellt.

Die Referenzierungseinheit 80 wird mit der Sägeschablone 14 gekoppelt und die Sägeschablone wiederum mit der Ausrichtvorrichtung 12 durch in Eingriff Bringen der Kopplungsglieder 20 und 22.

Der Operateur kann nun die in Figur 1 schematisch dargestellte Einheit umfassend die Referenzierungseinheit 180, die Sägeschablone 14 sowie die Ausrichteinrichtung 12 an den Knochen 18 heranführen und sich vom Navigationssystem 182 anzeigen lassen, ob er die gewünschte Schnittposition erreicht hat. Nach dieser Grobjustage hält der Operateur die Einheit an den Knochen 18 und legt diese durch Einführen des Befestigungselements 16 durch die Befestigungselementaufnahme 28 am Knochen 18 fest.

In einem nächsten Schritt wird nun der Winkel 44 mit Unterstützung des Navigationssystems 182 eingestellt. Hierfür wird das Einstellrad 56 entsprechend verdreht, bis die Neigung des ersten Kopplungsglieds 20 und damit auch des Führungsschlitzes 152 mit den präoperativ bestimmten Vorgaben übereinstimmt.

Nun kann abschließend eine Resektionshöhe eingestellt werden durch Verändern des Abstands 40. Dies wird wie beschrieben erreicht durch Verdrehen des Einstellrads 50 im Uhrzeigersinn oder im Gegenuhrzeigersinn.

Nun kann noch die gesamte Einheit um die vom Befestigungselement 16 definierte Längsachse verdreht werden, um einen Varus-Valgus-Winkel einzustellen. Auch dies erfolgt mit Unterstützung des Navigationssystems 182.

Hat der Operateur die gewünschte Position der Sägeschablone 14 gefunden, fixiert er diese mit weiteren, in den Figuren nicht dargestellten Befestigungselementen am Knochen 18, indem diese Befestigungselemente durch die Sägeschablonenbefestigungselementaufnahmen 74 hindurchgeführt und in den Knochen 18 eingetrieben werden.

Ist die Sägeschablone 14 hinreichend unbeweglich am Knochen 18 fixiert, kann das Befestigungselement 16 entfernt und danach auch die Ausrichteinrichtung 12 von der Sägeschablone 14 abgezogen werden, indem die ersten und zweiten Kopplungsglieder 20, 22 außer Eingriff gebracht werden.

Optional kann auch die Referenzierungseinheit 180 von der Sägeschablone 14 abgetrennt werden durch außer Eingriff Bringen der Kopplungselemente 176 und 178.

Mit der nun positionierten und fixierten Sägeschablone 14 kann der Operateur das Sägeblatt der chirurgischen Säge durch den Führungsschlitz 172 einführen und den Knochen 18 teilweise resezieren zur Vorbereitung einer Knochenfläche, an die ein Implantatteil angelegt und fixiert werden soll.

Das oben beschriebene Sägeschablonensystem 10 ermöglicht es insbesondere, eine exakte Positionierung mit einem möglichst kompakten Aufbau der Ausrichtvorrichtung 12 zu erreichen. Das Sägeschablonensystem 10 eignet sich daher insbesondere auch für minimalinvasive Eingriffe zur Implantation von Gelenkendoprothesen.

Die Umsetzung des Exzenterprinzips bei der Ausrichtvorrichtung 12 zum Einstellen sowohl des Winkel 44 als auch des Abstands 40, ermöglicht es insbesondere, vollständig auf Gewindetriebe und dergleichen zu verzichten. Trotzdem ist eine exakte und selbsthemmende Einstellung sowohl des Abstands 40 als auch des Winkels 44 möglich, und dies sogar ohne mehrere Umdrehungen der Einstellräder 50 und 56 vornehmen zu müssen. Eine präzise Justierung ist hier insbesondere durch Verdrehbewegungen im Bereich eines Umfangswinkels von etwa 90° möglich.

Da die Ausrichtvorrichtung zur Positionierung nur ein einziges Befestigungselement 60 benötigt, ist in der Regel keine zusätzliche oder größere Inzision als die, die für die Sägeschablone 14 sowieso benötigt wird, erforderlich.

### Bezugszeichenliste

- 10: Sägeschablonensystem
- 12: Ausrichtvorrichtung
- 14: Sägeschablone
- 16: Befestigungselement
- 18: Knochen
- 20: erstes Kopplungsglied
- 22: zweites Kopplungsglied
- 24: zweites Kopplungsglied
- 26: zweites Kopplungsglied
- 28: Befestigungselementaufnahme
- 30: Längsachse
- 32: Bohrung
- 34: Führungshülse
- 36: Kopplungsgliedlängsachse
- 38: Abstandsänderungseinrichtung
- 40: Abstand
- 42: Winkeländerungseinrichtung
- 44: Winkel
- 46: Abstandseinstellglieddrehachse
- 48: Abstandseinstellglied
- 50: Einstellrad
- 52: Winkeleinstellglieddrehachse
- 54: Winkeleinstellglied
- 56: Einstellrad
- 58: Grundkörper
- 60: Abstandseinstellgliedlagerelement
- 62: Schraube
- 64: Gewindebohrung
- 66: Durchbrechung
- 68: Winkeleinstellgliedlagerelement
- 70: Schraube
- 72: Gewindebohrung
- 74: Durchbrechung
- 76: Ausnehmung
- 78: Vorsprung
- 80: Schiebelement
- 82: Schiebelementlängsachse
- 84: Schiebelementführung
- 86: Durchbrechung
- 88: Bohrung
- 90: Aussparung
- 92: Bohrung
- 94: Ende
- 96: Bohrung
- 98: Hülse
- 100: Ende
- 102: Bohrung
- 104: Führungsstift
- 106: Ende
- 108: Schiebemitnehmer
- 110: Stift
- 112: Führungsausnehmung
- 114: Stift
- 116: Stift
- 118: Schwenkachse
- 120: Schwenkelement
- 122: Lagerbacken
- 124: Lagerstift
- 126: Schwenkmitnehmer
- 128: Stift
- 130: Schwenkmitnehmerführungsausnehmung
- 132: Abstandsschritteinstelleinrichtung
- 134: Abstandsrastmechanismus
- 136: erstes Abstandsrastglied
- 138: zweites Abstandsrastglied
- 140: Abstandsverzahnung
- 142: Zahn
- 144: Abstandsrastkörper
- 146: vorspannendes Element
- 148: Druckfeder
- 150: Seitenfläche
- 152: Querbohrung
- 154: Winkelschritteintelleinrichtung
- 156: Winkelrastmechanismus
- 158: erstes Winkelrastglied
- 160: zweites Winkelrastglied
- 162: Winkelverzahnung
- 164: Zahn
- 166: Winkelrastkörper
- 168: Seitenfläche
- 170: Führungsdurchbrechung
- 172: Führungsschlitz
- 174: Sägeschablonenbefestigungselementaufnahme
- 176: erstes Kopplungselement
- 178: zweites Kopplungselement
- 180: Referenzierungseinheit
- 182: Navigationssystem
- 184: Markerelement

## Patentansprüche

1. Medizinisches Sägeschablonensystem (10) umfassend eine Ausrichtvorrichtung (12) zum Ausrichten einer medizinischen Sägeschablone (14) relativ zu einem menschlichen oder tierischen Knochen (18), welche Ausrichtvorrichtung (12) mindestens eine Befestigungselementaufnahme (28) für ein in dem Knochen (18) festlegbares Befestigungselement (16), ein erstes Kopplungsglied (20), welches mit der medizinischen Sägeschablone (14) koppelbar oder gekoppelt ist, eine Abstandsänderungseinrichtung (38) zum Verändern eines Abstands (40) zwischen der Befestigungselementaufnahme (28) und dem ersten Kopplungsglied (20) und eine Winkeländerungseinrichtung (42) zum Verändern eines Winkels (44) zwischen der Befestigungselementaufnahme (28) und dem ersten Kopplungsglied (20) umfasst, wobei die Abstandsänderungseinrichtung (38) ein um eine Abstandseinstellglieddrehachse (46) verdrehbares Abstandseinstellglied (48) umfasst und wobei die Winkeländerungseinrichtung (42) ein um eine Winkeleinstellglieddrehachse (52) verdrehbares Winkeleinstellglied (54) umfasst, **dadurch gekennzeichnet, dass** die Abstandseinstellglieddrehachse (46) die Winkeleinstellglieddrehachse (52) definiert.

2. Medizinisches Sägeschablonensystem nach Anspruch 1, **dadurch gekennzeichnet, dass**
a) die Abstandsänderungseinrichtung (38) und die Winkeländerungseinrichtung (42) unabhängig voneinander betätigbar sind
und/oder
b) das Abstandseinstellglied (48) in Form eines Einstellrads (50) ausgebildet ist
und/oder
c) das Winkeleinstellglied (54) in Form eines Einstellrads (56) ausgebildet ist
und/oder
d) die Ausrichtvorrichtung (12) einen Grundkörper (58) umfasst, an welchem das Abstandseinstellglied (48) und das Winkeleinstellglied (54) verdrehbar gelagert oder gehalten sind
und/oder
e) dem Abstandseinstellglied (48) ein Abstandseinstellgliedlagerelement (60) zugeordnet ist und dass dem Winkeleinstellglied (54) ein Winkeleinstellgliedlagerelement (68) zugeordnet ist,
wobei insbesondere
e1) das Abstandseinstellgliedlagerelement (60) und/oder das Winkeleinstellgliedlagerelement (68) in Form eines Lagerstifts oder einer Schraube (70) ausgebildet sind
und/oder
e2) das Abstandseinstellgliedlagerelement (60) am Grundkörper (58) oder am Abstandseinstellglied (48) angeordnet oder ausgebildet ist
und/oder
e3) das Winkeleinstellgliedlagerelement (68) am Grundkörper (58) oder am Winkeleinstellglied (54) angeordnet oder ausgebildet ist,
und/oder
f) das Abstandseinstellglied (48) und/oder das Winkeleinstellglied (54) mindestens eine in radialer Richtung von der Abstandseinstellglieddrehachse (46) weg weisende Ausnehmung (76) und/oder mindestens einen in radialer Richtung von der Abstandseinstellglieddrehachse (46) weg weisenden Vorsprung (78) aufweisen.

3. Medizinisches Sägeschablonensystem nach einem der voranstehenden Ansprüche, **dadurch gekennzeichnet, dass** die Ausrichtvorrichtung (12) einen Grundkörper (58) umfasst, an welchem das Abstandseinstellglied (48) und das Winkeleinstellglied (54) verdrehbar gelagert oder gehalten sind, und dass die Abstandsänderungseinrichtung (38) ein am Grundkörper (58) verschiebbar gelagertes, eine Schiebeelementlängsachse (82) definierendes Schiebeelement (80) umfasst, welches mit dem Abstandseinstellglied (48) direkt oder indirekt zusammenwirkend angeordnet oder ausgebildet ist derart, dass das Schiebeelement (80) in Richtung der Schiebeelementlängsachse (82) verschiebbar ist durch Verdrehen des Abstandseinstellglieds um die Abstandseinstellglieddrehachse,
wobei insbesondere das Schiebeelement (80) am Grundkörper (58) bezogen auf die Schiebeelementlängsachse (82) unverdrehbar geführt ist.

4. Medizinisches Sägeschablonensystem nach Anspruch 3, **dadurch gekennzeichnet, dass** der Grundkörper (58) eine Schiebeelementführung (84) umfasst, in welcher das Schiebeelement (80) beweglich geführt ist.

5. Medizinisches Sägeschablonensystem nach Anspruch 4, **dadurch gekennzeichnet, dass**
a) die Schiebeelementführung (84) in Form einer Ausnehmung oder Durchbrechung (86) ausgebildet ist
und/oder
b) die Schiebeelementführung (84) einen unrunden Querschnitt aufweist
und/oder
c) die Abstandsänderungseinrichtung (38) einen exzentrisch zur Abstandseinstellglieddrehachse (46) angeordneten Schiebemitnehmer (108) umfasst, dass der Schiebemitnehmer (108) am Abstandseinstellglied (48) angeordnet oder ausgebildet ist und mit dem Schiebeelement (80) beweglich in Eingriff steht oder dass der Schiebemitnehmer (108) am Schiebeelement (80) angeordnet oder ausgebildet ist und mit dem Abstandseinstellglied (48) beweglich in Eingriff steht,
wobei insbesondere
c1) der Schiebemitnehmer (108) in Form eines Stifts (110) ausgebildet ist und dass eine Stiftlängsachse des Stifts (110) parallel oder im Wesentlichen parallel zur Abstandseinstellglieddrehachse (46) verläuft
und/oder
c2) der Schiebemitnehmer (108) in eine korrespondierende Führungsausnehmung (112) eingreift zum Übertragen einer Antriebskraft vom Abstandseinstellglied (48) auf das Schiebeelement (80).

6. Medizinisches Sägeschablonensystem nach einem der voranstehenden Ansprüche, **dadurch gekennzeichnet, dass** die Ausrichtvorrichtung (12) einen Grundkörper (58) umfasst, an welchem das Abstandseinstellglied (48) und das Winkeleinstellglied (54) verdrehbar gelagert oder gehalten sind, und dass die Winkeländerungseinrichtung (42) ein am Grundkörper (58) um eine Schwenkachse (118) verschwenkbar gelagertes Schwenkelement (120) umfasst, welches mit dem Winkeleinstellglied (54) direkt oder indirekt zusammenwirkend angeordnet oder ausgebildet ist derart, dass das Schwenkelement (120) um die Schwenkachse (118) verschwenkbar ist durch Verdrehen des Winkeleinstellglieds (54) um die Winkeleinstellglieddrehachse (52).

7. Medizinisches Sägeschablonensystem nach Anspruch 6, **dadurch gekennzeichnet, dass**
a) die Schwenkachse (118) parallel zur Winkeleinstellglieddrehachse (52) verläuft
und/oder
b) das erste Kopplungsglied (20) am Schwenkelement (120) angeordnet oder ausgebildet, insbesondere einstückig, ist
und/oder
c) das erste Kopplungsglied (20) eine Kopplungsgliedlängsachse (36) definiert und dass die Kopplungsgliedlängsachse (36) quer zur Schiebeelementlängsachse (82) verläuft,
wobei insbesondere die Kopplungsgliedlängsachse (36) quer, insbesondere senkrecht, zur Schwenkachse (118) verläuft,
und/oder
d) der Grundkörper (58) zwei Lagerbacken (122) umfasst, dass das Schwenkelement (120) zwischen den Lagerbacken (122) auf einem an den Lagerbacken (122) gehaltenen Lagerstift (124) gelagert ist oder dass die Lagerbacken auf zwei am Schwenkelement abstehenden Lagervorsprüngen gelagert sind,
wobei insbesondere der Lagerstift (124) oder die Lagervorsprünge die Schwenkachse (118) definieren.

8. Medizinisches Sägeschablonensystem nach Anspruch 6 oder 7, **dadurch gekennzeichnet, dass** die Winkeländerungseinrichtung (42) einen exzentrisch zur Winkeleinstellglieddrehachse (52) angeordneten Schwenkmitnehmer (126) umfasst, dass der Schwenkmitnehmer (126) am Winkeleinstellglied (54) angeordnet oder ausgebildet ist und mit dem Schwenkelement (120) beweglich in Eingriff steht oder dass der Schwenkmitnehmer am Schwenkelement angeordnet oder ausgebildet ist und mit dem Winkelseinstellglied beweglich in Eingriff steht,
wobei insbesondere
a) der Schwenkmitnehmer (126) in Form eines Stifts (128) ausgebildet ist und dass eine Stiftlängsachse des Stifts (128) parallel oder im Wesentlichen parallel zur Winkeleinstellglieddrehachse (52) verläuft
und/oder
b) der Schwenkmitnehmer (126) in eine korrespondierende Schwenkmitnehmerführungsausnehmung (130) eingreift zum Übertragen einer Antriebskraft vom Winkeleinstellglied (54) auf das Schwenkelement (120)
und/oder
c) die Schwenkachse (118) parallel oder im Wesentlichen parallel zur Abstandseinstellglieddrehachse (46) verläuft
und/oder
d) ein Abstand der Schwenkachse (118) von der Winkeleinstellglieddrehachse (52) größer ist als ein Abstand des Schwenkmitnehmers (126) von der Winkeleinstellglieddrehachse (52)
und/oder
e) ein Abstand des Schiebemitnehmers (108) von der Abstandseinstellglieddrehachse (46) größer ist als ein Abstand des Schwenkmitnehmers (126) von der Abstandseinstellglieddrehachse (46)
und/oder
f) ein Abstand der Schwenkachse (118) von der Winkeleinstellglieddrehachse (52) größer ist als ein Abstand der Schwenkachse (118) vom Schwenkmitnehmer (126).

9. Medizinisches Sägeschablonensystem nach einem der voranstehenden Ansprüche, **dadurch gekennzeichnet, dass**
a) die Abstandsänderungeinrichtung (38) ausgebildet ist zum Verändern des Abstands (40) zwischen der Befestigungselementaufnahme (28) und dem ersten Kopplungsglied (20) in diskreten Abstandsschritten,
wobei insbesondere die diskreten Abstandsschritte gleich groß oder im Wesentlichen gleich groß sind,
und/oder
b) die Abstandsänderungeinrichtung (38) eine Abstandsschritteinstelleinrichtung (132) umfasst zum Einstellen diskreter Abstände (40) zwischen der Befestigungselementaufnahme (28) und dem ersten Kopplungsglied (20),
wobei insbesondere die Abstandsschritteinstelleinrichtung (132) in Form eines Abstandsrastmechanismus (134) ausgebildet ist umfassend miteinander zusammenwirkende erste und zweite Abstandsrastglieder (136, 138) und wobei die ersten und zweiten Abstandsrastglieder (136, 138) bei diskreten Abständen (40) zwischen der Befestigungselementaufnahme (28) und dem ersten Kopplungsglied (20) in Eingriff stehen,
wobei weiter insbesondere
b1) die ersten und zweiten Abstandsrastglieder (136, 138) einerseits am Abstandseinstellglied (48) und andererseits am Grundkörper (58) angeordnet oder ausgebildet sind
und/oder
b2) eine Mehrzahl erster Abstandsrastglieder (136) in Form von eine Abstandsverzahnung (140) ausbildenden Zähne (142) ausgebildet ist, wobei insbesondere die Abstandsverzahnung (140) die Abstandseinstellglieddrehachse (46) konzentrisch umgebend angeordnet oder ausgebildet ist,
und/oder
b3) ein zweites Abstandsrastglied (138) in Form eines gegen ein vorspannendes Element (146) aus einer Grundstellung auslenkbaren Abstandsrastkörpers (144) ausgebildet ist oder
ein zweites Abstandsrastglied (138) in Form eines gegen ein vorspannendes Element (146) aus einer Grundstellung auslenkbaren Abstandsrastkörpers (144) ausgebildet ist und wobei eine Wirkrichtung des vorspannenden Elements (146) parallel oder im Wesentlichen parallel zur Abstandseinstellglieddrehachse (46) verläuft
und/oder
b4) die ersten Abstandsrastglieder (136) in Richtung auf den Grundkörper (58) hin weisend vom Abstandseinstellglied (48) abstehend angeordnet oder ausgebildet sind
und/oder
b5) ein Abstand der ersten Abstandsrastglieder (136) von der Abstandseinstellglieddrehachse (46) größer ist als ein Abstand des Schiebemitnehmers (108) von der Abstandseinstellglieddrehachse (46).

10. Medizinisches Sägeschablonensystem nach einem der voranstehenden Ansprüche, **dadurch gekennzeichnet, dass** die Winkeländerungseinrichtung (42) eine Winkelschritteinstelleinrichtung (154) umfasst zum Einstellen diskreter Winkel (44) zwischen der Befestigungselementaufnahme (28) und dem ersten Kopplungsglied (20),
wobei insbesondere die Winkelschritteinstelleinrichtung (154) in Form eines Winkelrastmechanismus (156) ausgebildet ist umfassend miteinander zusammenwirkende erste und zweite Winkelrastglieder (158, 160) und wobei die ersten und zweiten Winkelrastglieder(158, 160) bei diskreten Winkeln (44) zwischen der Befestigungselementaufnahme (28) und dem ersten Kopplungsglied (20) in Eingriff stehen,
wobei weiter insbesondere
a) die ersten und zweiten Winkelrastglieder (158, 160) einerseits am Winkeleinstellglied (54) und andererseits am Grundkörper (58) angeordnet oder ausgebildet sind
und/oder
b) eine Mehrzahl erster Winkelrastglieder (158) in Form von eine Winkelverzahnung (162) ausbildenden Zähne (164) ausgebildet ist oder
eine Mehrzahl erster Winkelrastglieder (158) in Form von eine Winkelverzahnung (162) ausbildenden Zähne (164) ausgebildet ist und die Winkelverzahnung (162) die Winkeleinstellglieddrehachse (52) konzentrisch umgebend angeordnet oder ausgebildet ist
und/oder
c) ein zweites Winkelrastglied (160) in Form eines gegen ein vorspannendes Element (146) aus einer Grundstellung auslenkbaren Winkelrastkörpers (166) ausgebildet ist
oder
ein zweites Winkelrastglied (160) in Form eines gegen ein vorspannendes Element (146) aus einer Grundstellung auslenkbaren Winkelrastkörpers (166) ausgebildet ist und wobei dem zweiten Abstandsrastglied (138) und dem zweiten Winkelrastglied (160) ein gemeinsames vorspannendes Element (146) zugeordnet ist
und/oder
d) ein zweites Abstandsrastglied (138) in Form eines gegen ein vorspannendes Element (146) aus einer Grundstellung auslenkbaren Abstandsrastkörpers (144) ausgebildet ist und dass eine Wirkrichtung des vorspannenden Elements (146) parallel oder im Wesentlichen parallel zur Winkeleinstellglieddrehachse (52) verläuft
und/oder
e) die ersten Winkelrastglieder (158) in Richtung auf den Grundkörper (58) hin weisend vom Winkeleinstellglied (54) abstehend angeordnet oder ausgebildet sind
und/oder
f) ein Abstand der ersten Winkelrastglieder (158) von der Winkeleinstellglieddrehachse (52) größer ist als ein Abstand des Schwenkmitnehmers (126) von der Winkeleinstellglieddrehachse (52).

11. Medizinisches Sägeschablonensystem nach einem der Ansprüche 3 bis 5, **dadurch gekennzeichnet, dass** die Befestigungselementaufnahme (28) am Schiebeelement (80) angeordnet oder ausgebildet ist, insbesondere in Form einer im Querschnitt rotationssymmetrischen Durchbrechung.

12. Medizinisches Sägeschablonensystem nach einem der voranstehenden Ansprüche, **gekennzeichnet durch** mindestens ein Befestigungselement (16) zum Verankern in einem Knochen (18),
wobei insbesondere das mindestens eine Befestigungselement (16) in Form eines Knochenpins oder in Form einer Knochenschraube ausgebildet ist.

13. Medizinisches Sägeschablonensystem nach einem der voranstehenden Ansprüche, **gekennzeichnet durch** eine Sägeschablone (14) mit mindestens einer Führungsdurchbrechung (170), insbesondere in Form eines Führungsschlitzes (172), für ein Sägeblatt.

14. Medizinisches Sägeschablonensystem nach Anspruch 13, **dadurch gekennzeichnet, dass**
a) die Sägeschablone (14) mindestens eine Sägeschablonenbefestigungselementaufnahme (174) zum Aufnehmen mindestens eines Befestigungselements umfasst
und/oder
b) die Sägeschablone (14) ein zweites Kopplungsglied (22) umfasst zum in Eingriff Bringen mit dem ersten Kopplungsglied (20)
und/oder
c) die Sägeschablone (14) mit dem ersten Kopplungsglied (20) unlösbar verbunden oder mit dem ersten Kopplungsglied (20) einstückig ausgebildet ist
und/oder
d) die Sägeschablone (14) mindestens ein erstes Kopplungselement (176) umfasst zum in Eingriff Bringen mit einem zweiten Kopplungselement (178) einer medizinischen Referenzierungseinheit (180).

15. Medizinisches Sägeschablonensystem nach einem der voranstehenden Ansprüche, **gekennzeichnet durch** eine medizinische Referenzierungseinheit (180), deren Position und/oder Orientierung im Raum mit einem medizinischen Navigationssystem (182) bestimmbar ist,
wobei insbesondere die Referenzierungseinheit (180) an der Sägeschablone (14) angeordnet oder ausgebildet ist oder wobei die Referenzierungseinheit (180) ein zweites Kopplungselement (178) umfasst zum in Eingriff Bringen mit dem ersten Kopplungselement (176) der Sägeschablone (14) in einer Kopplungsstellung.

## Claims

1. Medical sawing template system (10), comprising an aligning apparatus (12) for aligning a medical sawing template (14) relative to a human or animal bone (18), which aligning apparatus (12) comprises at least one fastening element receptacle (28) for a fastening element (16) fixable in the bone (18), a first coupling member (20) which is coupleable or coupled to the medical sawing template (14), a distance-changing device (38) for varying a distance (40) between the fastening element receptacle (28) and the first coupling member (20), and an angle-changing device (42) for varying an angle (44) between the fastening element receptacle (28) and the first coupling member (20), wherein the distance-changing device (38) comprises a distance-setting member (48) which is rotatable about a distance-setting member rotational axis (46), and wherein the angle-changing device (42) comprises an angle-setting member (54) which is rotatable about an angle-setting member rotational axis (52), **characterized in that** the distance-setting member rotational axis (46) defines the angle-setting member rotational axis (52).

2. Medical sawing template system in accordance with Claim 1, **characterized in that**
a) the distance-changing device (38) and the angle-changing device (42) are actuatable independently of each other
and/or
b) the distance-setting member (48) is configured in the form of a setting wheel (50)
and/or
c) the angle-setting member (54) is configured in the form of a setting wheel (56)
and/or
d) the aligning apparatus (12) comprises a base body (58) on which the distance-setting member (48) and the angle-setting member (54) are rotatably mounted or held
and/or
e) a distance-setting member bearing element (60) is associated with the distance-setting member (48), and **in that** an angle-setting member bearing element (68) is associated with the angle-setting member (54),
wherein, in particular,
e1) the distance-setting member bearing element (60) and/or the angle-setting member bearing element (68) are configured in the form of a bearing pin or a screw (70)
and/or
e2) the distance-setting member bearing element (60) is arranged or formed on the base body (58) or on the distance-setting member (48)
and/or
e3) the angle-setting member bearing element (68) is arranged or formed on the base body (58) or on the angle-setting member (54),
and/or
f) the distance-setting member (48) and/or the angle-setting member (54) have at least one recess (76) pointing in radial direction away from the distance-setting member rotational axis (46) and/or at least one projection (78) pointing in radial direction away from the distance-setting member rotational axis (46).

3. Medical sawing template system in accordance with any one of the preceding Claims, **characterized in that** the aligning apparatus (12) comprises a base body (58) on which the distance-setting member (48) and the angle-setting member (54) are rotatably mounted or held, and **in that** the distance-changing device (38) comprises a sliding element (80) which is displaceably mounted on the base body (58) and defines a sliding element longitudinal axis (82), which sliding element (80) is arranged or formed cooperating directly or indirectly with the distance-setting member (48) in such a way that the sliding element (80) is displaceable in the direction of the sliding element longitudinal axis (82) by rotating the distance-setting member about the distance-setting member rotational axis,
wherein, in particular, the sliding element (80) is guided on the base body (58) so as to be non-rotatable in relation to the sliding element longitudinal axis (82).

4. Medical sawing template system in accordance with Claim 3, **characterized in that** the base body (58) comprises a sliding element guide (84) in which the sliding element (80) is moveably guided.

5. Medical sawing template system in accordance with Claim 4, **characterized in that**
a) the sliding element guide (84) is configured in the form of a recess or perforation (86)
and/or
b) the sliding element guide (84) has a non-round cross section
and/or
c) the distance-changing device (38) comprises a sliding catch (108) arranged eccentrically to the distance-setting member rotational axis (46), **in that** the sliding catch (108) is arranged or formed on the distance-setting member (48) and is moveably in engagement with the sliding element (80), or **in that** the sliding catch (108) is arranged or formed on the sliding element (80) and is moveably in engagement with the distance-setting member (48),
wherein, in particular,
c1) the sliding catch (108) is configured in the form of a pin (110), and **in that** a pin longitudinal axis of the pin (110) runs parallel or substantially parallel to the distance-setting member rotational axis (46)
and/or
c2) the sliding catch (108) engages into a corresponding guide recess (112) for transmitting a drive force from the distance-setting member (48) to the sliding element (80).

6. Medical sawing template system in accordance with any one of the preceding Claims, **characterized in that** the aligning apparatus (12) comprises a base body (58) on which the distance-setting member (48) and the angle-setting member (54) are rotatably mounted or held, and **in that** the angle-changing device (42) comprises a pivot element (120) which is mounted on the base body (58) so as to be pivotable about a pivot axis (118), which pivot element (120) is arranged or formed cooperating directly or indirectly with the angle-setting member (54) in such a way that the pivot element (120) is pivotable about the pivot axis (118) by rotating the angle-setting member (54) about the angle-setting member rotational axis (52).

7. Medical sawing template system in accordance with Claim 6, **characterized in that**
a) the pivot axis (118) runs parallel to the angle-setting member rotational axis (52)
and/or
b) the first coupling member (20) is arranged or formed, in particular in one piece, on the pivot element (120)
and/or
c) the first coupling member (20) defines a coupling member longitudinal axis (36), and **in that** the coupling member longitudinal axis (36) runs transversely to the sliding element longitudinal axis (82), wherein, in particular, the coupling member longitudinal axis (36) runs transversely, in particular perpendicularly, to the pivot axis (118),
and/or
d) the base body (58) comprises two bearing jaws (122), **in that** the pivot element (120) is mounted between the bearing jaws (122) on a bearing pin (124) held on the bearing jaws (122), or **in that** the bearing jaws are mounted on two bearing projections projecting on the pivot element,
wherein, in particular, the bearing pin (124) or the bearing projections define the pivot axis (118).

8. Medical sawing template system in accordance with Claim 6 or 7, **characterized in that** the angle-changing device (42) comprises a pivoting catch (126) arranged eccentrically to the angle-setting member rotational axis (52), **in that** the pivoting catch (126) is arranged or formed on the angle-setting member (54) and is moveably in engagement with the pivot element (120), or **in that** the pivoting catch is arranged or formed on the pivot element and is moveably in engagement with the angle-setting member,
wherein, in particular,
a) the pivoting catch (126) is configured in the form of a pin (128), and **in that** a pin longitudinal axis of the pin (128) runs parallel or substantially parallel to the angle-setting member rotational axis (52)
and/or
b) the pivoting catch (126) engages into a corresponding pivoting catch guide recess (130) for transmitting a drive force from the angle-setting member (54) to the pivoting element (120)
and/or
c) the pivot axis (118) runs parallel or substantially parallel to the distance-setting member rotational axis (46)
and/or
d) a distance of the pivot axis (118) from the angle-setting member rotational axis (52) is greater than a distance of the pivoting catch (126) from the angle-setting member rotational axis (52)
and/or
e) a distance of the sliding catch (108) from the distance-setting member rotational axis (46) is greater than a distance of the pivoting catch (126) from the distance-setting member rotational axis (46)
and/or
f) a distance of the pivot axis (118) from the angle-setting member rotational axis (52) is greater than a distance of the pivot axis (118) from the pivoting catch (126).

9. Medical sawing template system in accordance with any one of the preceding Claims, **characterized in that**
a) the distance-changing device (38) is configured for varying the distance (40) between the fastening element receptacle (28) and the first coupling member (20) in discrete distance steps,
wherein, in particular, the discrete distance steps are the same size or substantially the same size,
and/or
b) the distance-changing device (38) comprises a distance step-setting device (132) for setting discrete distances (40) between the fastening element receptacle (28) and the first coupling member (20),
wherein, in particular, the distance step-setting device (132) is configured in the form of a distance latching mechanism (134), comprising mutually cooperating first and second distance latching members (136, 138), and wherein the first and second distance latching members (136, 138) are in engagement at discrete distances (40) between the fastening element receptacle (28) and the first coupling member (20),
wherein, further in particular,
b1) the first and second distance latching members (136, 138) are arranged or formed on the distance-setting member (48) on the one hand and on the base body (58) on the other hand
and/or
b2) a plurality of first distance latching members (136) is configured in the form of teeth (142) forming a distance toothing (140), wherein, in particular, the distance toothing (140) is arranged or formed concentrically surrounding the distance-setting member rotational axis (46),
and/or
b3) a second distance latching member (138) is configured in the form of a distance latching body (144) which is deflectable out of a base position against a biasing element (146)
or
a second distance latching member (138) is configured in the form of a distance latching body (144) which is deflectable out of a base position against a biasing element (146), and wherein a direction of action of the biasing element (146) runs parallel or substantially parallel to the distance-setting member rotational axis (46)
and/or
b4) the first distance latching members (136) are arranged or formed projecting from the distance-setting member (48) pointing in the direction toward the base body (58)
and/or
b5) a distance of the first distance latching members (136) from the distance-setting member rotational axis (46) is greater than a distance of the sliding catch (108) from the distance-setting member rotational axis (46).

10. Medical sawing template system in accordance with any one of the preceding Claims, **characterized in that** the angle-changing device (42) comprises an angle step-setting device (154) for setting discrete angles (44) between the fastening element receptacle (28) and the first coupling member (20),
wherein, in particular, the angle step-setting device (154) is configured in the form of an angle latching mechanism (156) comprising mutually cooperating first and second angle latching members (158, 160), and wherein the first and second angle latching members (158, 160) are in engagement at discrete angles (44) between the fastening element receptacle (28) and the first coupling member (20),
wherein, further in particular,
a) the first and second angle latching members (158, 160) are arranged or formed on the angle-setting member (54) on the one hand and on the base body (58) on the other hand
and/or
b) a plurality of first angle latching members (158) is configured in the form of teeth (164) forming an angle toothing (162)
or
a plurality of first angle latching members (158) is configured in the form of teeth (164) forming an angle toothing (162), and the angle toothing (162) is arranged or formed concentrically surrounding the angle-setting member rotational axis (52)
and/or
c) a second angle latching member (160) is configured in the form of an angle latching body (166) which is deflectable out of a base position against a biasing element (146)
or
a second angle latching member (160) is configured in the form of an angle latching body (166) which is deflectable out of a base position against a biasing element (146), and wherein a common biasing element (146) is associated with the second distance latching member (138) and the second angle latching member (160)
and/or
d) a second distance latching member (138) is configured in the form of a distance latching body (144) which is deflectable out of a base position against a biasing element (146), and wherein a direction of action of the biasing element (146) runs parallel or substantially parallel to the angle-setting member rotational axis (52)
and/or
e) the first distance latching members (158) are arranged or formed projecting from the angle-setting member (54) pointing in the direction toward the base body (58)
and/or
f) a distance of the first angle latching members (158) from the angle-setting member rotational axis (52) is greater than a distance of the pivoting catch (126) from the angle-setting member rotational axis (52).

11. Medical sawing template system in accordance with any one of Claims 3 to 5, **characterized in that** the fastening element receptacle (28) is arranged or formed on the sliding element (80), in particular in the form of a perforation which is rotationally symmetrical in cross section.

12. Medical sawing template system in accordance with any one of the preceding Claims, **characterized by** at least one fastening element (16) for anchoring in a bone (18),
wherein, in particular, the at least one fastening element (16) is configured in the form of a bone pin or in the form of a bone screw.

13. Medical sawing template system in accordance with any one of the preceding Claims, **characterized by** a sawing template (14) with at least one guide perforation (170), in particular in the form of a guide slot (172), for a saw blade.

14. Medical sawing template system in accordance with Claim 13, **characterized in that**
a) the sawing template (14) comprises at least one sawing template fastening element receptacle (174) for accommodating at least one fastening element
and/or
b) the sawing template (14) comprises a second coupling member (22) for bringing into engagement with the first coupling member (20)
and/or
c) the sawing template (14) is non-releasably connected to the first coupling member (20) or is formed as one piece with the first coupling member (20)
and/or
d) the sawing template (14) comprises at least one first coupling element (176) for bringing into engagement with a second coupling element (178) of a medical referencing unit (180).

15. Medical sawing template system in accordance with any one of the preceding Claims, **characterized in that** the medical sawing template system comprises a medical referencing unit (180), the position and the orientation in space of which is determinable with a medical navigation system (182),
wherein, in particular, the medical referencing unit (180) is arranged or formed on the sawing template (14), or wherein the referencing unit (180) comprises a second coupling element (178) for bringing into engagement with the first coupling element (176) of the sawing template (14) in a coupling position.

## Revendications

1. Système de gabarits de coupe (10) médical comprenant un dispositif d'orientation (12) pour l'orientation d'un gabarit de coupe (14) médical par rapport à un os (18) humain ou animal, lequel dispositif d'orientation (12) comprend au moins une réception d'élément de fixation (28) pour un élément de fixation (16) pouvant être fixé dans l'os (18), un premier organe d'accouplement (20), lequel peut être ou est couplé avec le gabarit de coupe (14) médical, un équipement de modification de distance (38) pour la modification d'une distance (40) entre la réception d'élément de fixation (28) et le premier organe d'accouplement (20) et un équipement de modification d'angle (42) pour la modification d'un angle (44) entre la réception d'élément de fixation (28) et le premier organe d'accouplement (20), dans lequel l'équipement de modification de distance (38) comprend un organe de réglage de distance (48) pouvant être tourné autour d'un axe de rotation d'organe de réglage de distance (46) et dans lequel l'équipement de modification d'angle (42) comprend un organe de réglage d'angle (54) pouvant être tourné autour d'un axe de rotation d'organe de réglage d'angle (52), **caractérisé en ce que** l'axe de rotation d'organe de réglage de distance (46) définit l'axe de rotation d'organe de réglage d'angle (52).

2. Système de gabarits de coupe médical selon la revendication 1, **caractérisé en ce que**
a) l'équipement de modification de distance (38) et l'équipement de modification d'angle (42) peuvent être actionnés indépendamment l'un de l'autre
et/ou
b) l'organe de réglage de distance (48) est conçu sous la forme d'une roue de réglage (50)
et/ou
c) l'organe de réglage d'angle (54) est conçu sous la forme d'une roue de réglage (56)
et/ou
d) le dispositif d'orientation (12) comprend un corps de base (58) contre lequel l'organe de réglage de distance (48) et l'organe de réglage d'angle (54) sont montés ou maintenus en rotation
et/ou
e) un élément de support d'organe de réglage de distance (60) est attribué à l'organe de réglage de distance (48) et **en ce qu'**un élément de support d'organe de réglage d'angle (68) est attribué à l'organe de réglage d'angle (54),
dans lequel en particulier
e1) l'élément de support d'organe de réglage de distance (60) et/ou l'élément de support d'organe de réglage d'angle (68) sont conçus sous la forme d'une broche de support ou d'une vis (70)
et/ou
e2) l'élément de support d'organe de réglage de distance (60) est agencé ou conçu contre le corps de base (58) ou contre l'organe de réglage de distance (48)
et/ou
e3) l'élément de support d'organe de réglage d'angle (68) est agencé ou conçu contre le corps de base (58) ou contre l'organe de réglage d'angle (54),
et/ou
f) l'organe de réglage de distance (48) et/ou l'organe de réglage d'angle (54) présentent au moins un évidement (76) pointant dans la direction radiale en s'éloignant de l'axe de rotation d'organe de réglage de distance (46) et/ou au moins une saillie (78) pointant dans la direction radiale en s'éloignant de l'axe de rotation d'organe de réglage de distance (46).

3. Système de gabarits de coupe médical selon l'une des revendications précédentes, **caractérisé en ce que** le dispositif d'orientation (12) comprend un corps de base (58) contre lequel l'organe de réglage de distance (48) et l'organe de réglage d'angle (54) sont montés ou maintenus en rotation, et **en ce que** l'équipement de modification de distance (38) comprend un élément poussoir (80) monté de façon à pouvoir être poussé contre le corps de base (58) et définissant un axe longitudinal d'élément poussoir (82), lequel élément poussoir est agencé ou conçu en coopération directe ou indirecte avec l'organe de réglage de distance (48) de sorte que l'élément poussoir (80) peut être poussé dans la direction de l'axe longitudinal d'élément poussoir (82) par rotation de l'organe de réglage de distance autour de l'axe de rotation d'organe de réglage de distance,
dans lequel en particulier l'élément poussoir (80) est guidé contre le corps de base (58) de façon à ne pas pouvoir tourner par rapport à l'axe longitudinal d'élément poussoir (82).

4. Système de gabarits de coupe médical selon la revendication 3, **caractérisé en ce que** le corps de base (58) comprend un guidage d'élément poussoir (84) dans lequel l'élément poussoir (80) est guidé de façon mobile.

5. Système de gabarits de coupe médical selon la revendication 4, **caractérisé en ce que**
a) le guidage d'élément poussoir (84) est conçu sous la forme d'un évidement ou d'un perçage (86)
et/ou
b) le guidage d'élément poussoir (84) présente une section transversale non circulaire
et/ou
c) l'équipement de modification de distance (38) comprend un entraîneur de poussoir (108) agencé de façon excentrique par rapport à l'axe de rotation d'organe de réglage de distance (46), **en ce que** l'entraîneur de poussoir (108) est agencé ou conçu contre l'organe de réglage de distance (48) et est en prise de façon mobile avec l'élément poussoir (80) ou **en ce que** l'entraîneur de poussoir (108) est agencé ou conçu contre l'élément poussoir (80) et est en prise de façon mobile avec l'organe de réglage de distance (48),
dans lequel en particulier
c1) l'entraîneur de poussoir (108) est conçu sous la forme d'une broche (110) et **en ce qu'**un axe longitudinal de broche de la broche (110) se déroule parallèlement ou essentiellement parallèlement à l'axe de rotation d'organe de réglage de distance (46)
et/ou
c2) l'entraîneur de poussoir (108) vient en prise dans un évidement de guidage (112) correspondant pour la transmission d'une force d'entraînement de l'organe de réglage de distance (48) à l'élément poussoir (80).

6. Système de gabarits de coupe médical selon l'une des revendications précédentes, **caractérisé en ce que** le dispositif d'orientation (12) comprend un corps de base (58) contre lequel l'organe de réglage de distance (48) et l'organe de réglage d'angle (54) sont montés ou maintenus en rotation, et **en ce que** l'équipement de modification d'angle (42) comprend un élément de pivotement (120) monté en pivotement contre le corps de base (58) autour d'un axe de pivotement (118), lequel élément de pivotement est agencé ou conçu en coopération directe ou indirecte avec l'organe de réglage d'angle (54) de sorte que l'élément de pivotement (120) puisse être en pivotement autour de l'axe de pivotement (118) par rotation de l'organe de réglage d'angle (54) autour de l'axe de rotation d'organe de réglage d'angle (52).

7. Système de gabarits de coupe médical selon la revendication 6, **caractérisé en ce que**
a) l'axe de pivotement (118) se déroule parallèlement à l'axe de rotation d'organe de réglage d'angle (52)
et/ou
b) le premier organe d'accouplement (20) est agencé ou conçu contre l'élément de pivotement (120), en particulier en une seule pièce,
et/ou
c) le premier organe d'accouplement (20) définit un axe longitudinal d'organe d'accouplement (36) et **en ce que** l'axe longitudinal d'organe d'accouplement (36) se déroule transversalement à l'axe longitudinal d'élément poussoir (82),
dans lequel en particulier l'axe longitudinal d'organe d'accouplement (36) se déroule transversalement, en particulier perpendiculairement, à l'axe de pivotement (118),
et/ou
d) le corps de base (58) comprend deux mâchoires de support (122), **en ce que** l'élément de pivotement (120) est monté entre les mâchoires de support (122) sur une broche de support (124) maintenue contre les mâchoires de support (122) ou **en ce que** les mâchoires de support sont montées sur deux saillies de support dépassant au niveau de l'élément de pivotement,
dans lequel en particulier la broche de support (124) ou les saillies de support définissent l'axe de pivotement (118).

8. Système de gabarits de coupe médical selon la revendication 6 ou 7, **caractérisé en ce que** l'équipement de modification d'angle (42) comprend un entraîneur de pivotement (126) agencé de façon excentrique par rapport à l'axe de rotation d'organe de réglage d'angle (52), **en ce que** l'entraîneur de pivotement (126) est agencé ou conçu contre l'organe de réglage d'angle (54) et est en prise de façon mobile avec l'élément de pivotement (120) ou **en ce que** l'entraîneur de pivotement est agencé ou conçu contre l'élément de pivotement et est en prise de façon mobile avec l'organe de réglage d'angle,
dans lequel en particulier
a) l'entraîneur de pivotement (126) est conçu sous la forme d'une broche (128) et **en ce qu'**un axe longitudinal de broche de la broche (128) se déroule parallèlement ou essentiellement parallèlement à l'axe de rotation d'organe de réglage d'angle (52)
et/ou
b) l'entraîneur de pivotement (126) vient en prise dans un évidement de guidage d'entraîneur de pivotement (130) correspondant pour la transmission d'une force d'entraînement de l'organe de réglage d'angle (54) à l'élément de pivotement (120)
et/ou
c) l'axe de pivotement (118) se déroule parallèlement ou essentiellement parallèlement à l'axe de rotation d'organe de réglage de distance (46)
et/ou
d) une distance de l'axe de pivotement (118) depuis l'axe de rotation d'organe de réglage d'angle (52) est supérieure à une distance de l'entraîneur de pivotement (126) depuis l'axe de rotation d'organe de réglage d'angle (52)
et/ou
e) une distance de l'entraîneur de poussoir (108) depuis l'axe de rotation d'organe de réglage de distance (46) est supérieure à une distance de l'entraîneur de pivotement (126) depuis l'axe de rotation d'organe de réglage de distance (46)
et/ou
f) une distance de l'axe de pivotement (118) depuis l'axe de rotation d'organe de réglage d'angle (52) est supérieure à une distance de l'axe de pivotement (118) depuis l'entraîneur de pivotement (126).

9. Système de gabarits de coupe médical selon l'une des revendications précédentes, **caractérisé en ce que**
a) l'équipement de modification de distance (38) est conçu pour une modification de la distance (40) entre la réception d'élément de fixation (28) et le premier organe d'accouplement (20) en des pas de distance discrets,
dans lequel en particulier les pas de distance discrets sont de même taille ou essentiellement de même taille,
et/ou
b) l'équipement de modification de distance (38) comprend un équipement de réglage de pas de distance (132) pour le réglage de distances (40) discrètes entre la réception d'élément de fixation (28) et le premier organe d'accouplement (20),
dans lequel en particulier l'équipement de réglage de pas de distance (132) est conçu sous la forme d'un mécanisme à cliquet de distance (134) comprenant des premiers et seconds organes de cliquet de distance (136, 138) coopérant ensemble et dans lequel les premiers et seconds organes de cliquet de distance (136, 138) sont en prise à des distances (40) discrètes entre la réception d'élément de fixation (28) et le premier organe d'accouplement (20),
dans lequel en outre en particulier
b1) les premiers et seconds organes de cliquet de distance (136, 138) sont agencés ou conçus d'un côté contre l'organe de réglage de distance (48) et de l'autre côté contre le corps de base (58)
et/ou
b2) une pluralité de premiers organes de cliquet de distance (136) sont conçus sous la forme de dents (142) formant une denture de distance (140), dans lequel en particulier la denture de distance (140) est agencée ou conçue de façon à entourer l'axe de rotation d'organe de réglage de distance (46) de façon concentrique,
et/ou
b3) un second organe de cliquet de distance (138) est conçu sous la forme d'un corps de cliquet de distance (144) pouvant être dévié contre un élément de précontrainte (146) depuis une position de base
ou
un second organe de cliquet de distance (138) est conçu sous la forme d'un corps de cliquet de distance (144) pouvant être dévié contre un élément de précontrainte (146) depuis une position de base et dans lequel une direction d'action de l'élément de précontrainte (146) se déroule parallèlement ou essentiellement parallèlement à l'axe de rotation d'organe de réglage de distance (46)
et/ou
b4) les premiers organes de cliquet de distance (136) sont agencés ou conçus de façon à dépasser depuis l'organe de réglage de distance (48) dans la direction pointant vers le corps de base (58)
et/ou
b5) une distance des premiers organes de cliquet de distance (136) depuis l'axe de rotation d'organe de réglage de distance (46) est supérieure à une distance de l'entraîneur de poussoir (108) depuis l'axe de rotation d'organe de réglage de distance (46).

10. Système de gabarits de coupe médical selon l'une des revendications précédentes, **caractérisé en ce que** l'équipement de modification d'angle (42) comprend un équipement de réglage de pas angulaire (154) pour le réglage d'angles (44) discrets entre la réception d'élément de fixation (28) et le premier organe d'accouplement (20),
dans lequel en particulier l'équipement de réglage de pas angulaire (154) est conçu sous la forme d'un mécanisme à cliquet d'angle (156) comprenant des premiers et seconds organes de cliquet d'angle (158, 160) coopérant ensemble et dans lequel les premiers et seconds organes de cliquet d'angle (158, 160) sont en prise à des angles (44) discrets entre la réception d'élément de fixation (28) et le premier organe d'accouplement (20),
dans lequel en outre en particulier
a) les premiers et seconds organes de cliquet d'angle (158, 160) sont agencés ou conçus d'un côté contre l'organe de réglage d'angle (54) et de l'autre côté contre le corps de base (58)
et/ou
b) une pluralité de premiers organes de cliquet d'angle (158) est conçue sous la forme de dents (164) formant une denture d'angle (162)
ou
une pluralité de premiers organes de cliquet d'angle (158) est conçue sous la forme de dents (164) formant une denture d'angle (162) et la denture d'angle (162) est agencée ou conçue de façon à entourer l'axe de rotation d'organe de réglage d'angle (52) de façon concentrique,
et/ou
c) un second organe de cliquet d'angle (160) est conçu sous la forme d'un corps de cliquet d'angle (166) pouvant être dévié contre un élément de précontrainte (146) depuis une position de base
ou
un second organe de cliquet d'angle (160) est conçu sous la forme d'un corps de cliquet d'angle (166) pouvant être dévié contre un élément de précontrainte (146) depuis une position de base et dans lequel un élément de précontrainte (146) commun est attribué au second organe de cliquet de distance (138) et au second organe de cliquet d'angle (160)
et/ou
d) un second organe de cliquet de distance (138) est conçu sous la forme d'un corps de cliquet de distance (144) pouvant être dévié contre un élément de précontrainte (146) depuis une position de base et dans lequel une direction d'action de l'élément de précontrainte (146) se déroule parallèlement ou essentiellement parallèlement à l'axe de rotation d'organe de réglage d'angle (52)
et/ou
e) les premiers organes de cliquet d'angle (158) sont agencés ou conçus de façon à dépasser depuis l'organe de réglage d'angle (54) dans la direction pointant vers le corps de base (58)
et/ou
f) une distance des premiers organes de cliquet d'angle (158) depuis l'axe de rotation d'organe de réglage d'angle (52) est supérieure à une distance de l'entraîneur de pivotement (126) depuis l'axe de rotation d'organe de réglage d'angle (52).

11. Système de gabarits de coupe médical selon l'une des revendications précédentes 3 à 5, **caractérisé en ce que** la réception d'élément de fixation (28) est agencée ou conçue contre l'élément poussoir (80),
en particulier sous la forme d'un perçage symétrique en rotation en section transversale.

12. Système de gabarits de coupe médical selon l'une des revendications précédentes, **caractérisé par** au moins un élément de fixation (16) pour l'ancrage dans un os (18),
dans lequel en particulier le au moins un élément de fixation (16) est conçu sous la forme d'une broche pour os ou sous la forme d'une vis pour os.

13. Système de gabarits de coupe médical selon l'une des revendications précédentes, **caractérisé par** un gabarit de coupe (14) avec au moins un perçage de guidage (170), en particulier sous la forme d'une fente de guidage (172), pour une lame de coupe.

14. Système de gabarits de coupe médical selon la revendication 13, **caractérisé en ce que**
a) le gabarit de coupe (14) comprend au moins une réception d'élément de fixation de gabarit de coupe (174) pour la réception d'au moins un élément de fixation
et/ou
b) le gabarit de coupe (14) comprend un second organe d'accouplement (22) pour l'amenée en prise avec le premier organe d'accouplement (20)
et/ou
c) le gabarit de coupe (14) est relié de façon inamovible avec le premier organe d'accouplement (20) ou est conçu en une seule pièce avec le premier organe d'accouplement (20)
et/ou
d) le gabarit de coupe (14) comprend au moins un premier élément d'accouplement (176) pour l'amenée en prise avec un second élément d'accouplement (178) d'une unité de référencement (180) médicale.

15. Système de gabarits de coupe médical selon l'une des revendications précédentes, **caractérisé par** une unité de référencement (180) médicale dont la position et/ou l'orientation dans l'espace peut être déterminée avec un système de navigation médical (182),
dans lequel en particulier l'unité de référencement (180) est agencée ou conçue au niveau du gabarit de coupe (14) ou dans lequel l'unité de référencement (180) comprend un second élément d'accouplement (178) pour l'amenée en prise avec le premier élément d'accouplement (176) du gabarit de coupe (14) dans une position d'accouplement.
